(19) Europäisches Patentamt
European Patent Office
Office européen des brevets

(11) Publication number: **0 081 783**
**B2**

# (12) NEW EUROPEAN PATENT SPECIFICATION

(45) Date of publication of the new patent specification: **08.02.89**

(51) Int. Cl.⁴: **C 07 K 7/06**, A 61 K 37/02, A 61 K 37/64

(21) Application number: **82111259.6**

(22) Date of filing: **06.12.82**

(54) **Renin inhibitory peptides having Phe 13 deletion.**

(30) Priority: **10.12.81 US 329166**

(43) Date of publication of application: **22.06.83 Bulletin 83/25**

(45) Publication of the grant of the patent: **19.06.85 Bulletin 85/25**

(45) Mention of the opposition decision: **08.02.89 Bulletin 89/6**

(84) Designated Contracting States: **AT BE CH DE FR GB IT LI LU NL SE**

(56) References cited:
**FR-A- 2 262 532**

**H.UMEZAWA et al.: "Bioactive peptides produced by microorganisms", John Wiley & Sons, 1978, pages 137-141,149-150, New York (USA);**
**JOURNAL OF MEDICINAL CHEMISTRY, vol. 22, no. 5, 1979, pages 577-579, American Chemical Society; W.-S.LIU et al.: "Synthesis of all the stereoisomers of statine (4-Amino-3-hydroxy-6-methylheptanoic acid). Inhibition of pepsin activity N-carbobenzoxy-L-valyl-L-valyl-statine derived from the four stereoisomers".**

(73) Proprietor: **MERCK & CO. INC., 126, East Lincoln Avenue P.O. Box 2000, Rahway New Jersey 07065-0900 (US)**

(72) Inventor: **Boger, Joshua S., 719 New Gulph Road, Bryn Mawr Pennsylvania 19010 (US)**
Inventor: **Veber, Daniel F., 290 Batleson Road, Ambler Pennsylvania 19002 (US)**
Inventor: **Bock, Mark G., 1603 Leon Drive, Hatfield Pennsylvania 19440 (US)**

(74) Representative: **Abitz, Walter, Dr.-Ing. et al, Abitz, Morf, Gritschneder, Freiherr von Wittgenstein Postfach 86 01 09, D-8000 München 86 (DE)**

EP 0 081 783 B2

## Description

Background of the Invention

1. Field of the Invention

The present invention is concerned with peptides which inhibit renin.

The present invention is also concerned with pharmaceutical compositions containing the peptides of the present invention as active ingredients, with methods of treating renin-associated hypertension and hyperaldosteronism, with diagnostic methods which utilize the novel peptides of the present invention.

Renin is a proteolytic enzyme of molecular weight about 40,000, produced and secreted by the kidney. It is secreted by the juxtaglomerular cells and acts on the plasma substrate, angiotensinogen, to split off the decapeptide angiotensin I, which is converted to the potent pressor agent angiotensin II. Thus, the renin-angiotensin system plays an important role in normal cardiovascular homeostasis and in some forms of hypertension.

In the past, attempts to modulate or manipulate the renin-angiotensin system have met with success in the use of inhibitors of angiotensin I converting enzyme. In view of this success, it seems reasonable to conclude that a specific inhibitor of the limiting enzymatic step that ultimately regulates angiotensin II production, the action of renin on its substrate, would be at least equally successful. Thus, an effective inhibitor of renin has been long sought as both a therapeutic agent and as an investigative tool.

2. Brief Description of the Prior Art

There has been substantial interest in the synthesis of useful renin inhibitors for many decades; and the following table lists the major classes of renin inhibitors that have been studied, as well as their inhibition constants ($K_i$):

| Class | $K_i$(M) |
|---|---|
| Renin antibody | probably $10^{-6}$ |
| Pepstatin | $10^{-6}$–$10^{-7}$ |
| Phospholipids | $10^{-3}$ |
| Substrate analogs | |
| Tetrapeptides | $10^{-3}$ |
| Octa- to tridecapeptides | $10^{-5}$–$10^{-6}$ |

Umezawa et al., in J. Antibiot. (Tokyo) 23:259–262, 1970, reported the isolation of a peptide from actinomyces that was an inhibitor of aspartyl proteases such as pepsin, cathepsin D, and renin. This peptide, known as pepstatin, was found by Gross et al., Science 175:656, 1971, to reduce blood pressure in vivo after the injection of hog renin into nephrectomized rats. However, pepstatin has not found wide application as an experimental agent because of its limited solubility and its inhibition of a variety of other acid proteases in addition to renin. The structure of pepstatin is shown below:

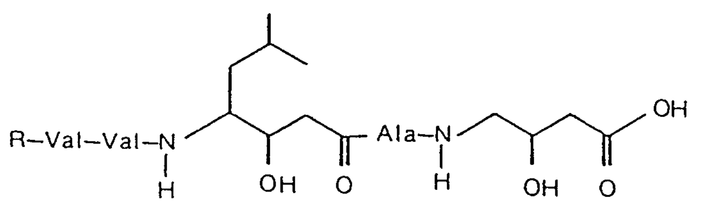

To date, many efforts have been made to prepare a specific renin inhibitor based on substrate analogy. Since the human renin substrate has only recently been elucidated (Tewksbury et al., Circulation 59, 60, Supp. II: 132, Oct. 1979), heretofore substrate analogy has been based on the known pig renin substrate. While the human and pig renin substrates are not the same, the substrate analogy based on pig renin has always been considered acceptable in the art as predictive of human renin inhibitory activity because of the closely related activity of the two renins. Thus, while pig renin does not cleave the human renin substrate, human renin, on the other hand, does cleave the pig renin substrate. See Poulsen et al., Biochim. Biophys. Acta 452:533–537, 1976; and Skeggs, Jr. et al., J. Exp. Med. 106:439–453, 1957. Moreover, the human renin inhibitory activity of the peptides of the present invention most active in inhibiting pig renin has been confirmed, thus providing further evidence of this accepted correlation between human and pig renin activity.

It has been found, for example, using pig renin substrate analogy, that the octapeptide sequence extending from histidine-6 through tyrosine-13 has kinetic parameters essentially the same as those of the full tetradecapeptide renin substrate. The amino acid sequence of the octapeptide in pig renin substrate is as follows:

| 6 | 7 | 8 | 9 | 10 | 11 | 12 | 13 |
|---|---|---|---|---|---|---|---|
| -His | -Pro | -Phe | -His | -Leu | -Leu | -Val | -Tyr- |

Renin cleaves this substrate between $Leu^{10}$ and $Leu^{11}$.

Kokubu et al., Biochem. Pharmacol. 22: 3217–3223, 1973, synthesized a number of analogs of the tetrapeptide found between residues 10 to 13, but while inhibition could be shown, inhibitory constants were only of the order of $10^{-3}$M.

Analogs of a larger segment of renin substrate were also synthesized: Burton et al., Biochemistry 14: 3892–3898, 1975, and Poulsen et al., Biochemistry 12: 3877–3882, 1973. Two of the major

obstacles which had to be overcome to obtain an effective renin inhibitor useful in vivo were lack of solubility and weak binding (large inhibitory constant). Modifications to increase solubility soon established that the inhibitory properties of the peptides are markedly dependent on the hydrophobicity of various amino acid residues, and that increasing solubility by replacing lipophillic amino acids with hydrophilic isosteric residues becomes counterproductive. Other approaches to increasing solubility have had limited success. Various modifications designed to increase binding to renin have also been made, but here too, with only limited success. For a more detailed description of past efforts to prepare an effective inhibitor of renin, see Haber and Burton, Fed. Proc. Fed. Am. Soc. Exp. Biol. 38: 2768-2773, 1979.

For other articles describing previous efforts to devise renin inhibitors, see Marshall, Federation Proc. 35: 2494-2501, 1976, Burton et al., Proc. Natl. Acad. Sci. USA 77: 5476-5479, Sept. 1980; Suketa et al., Biochemistry 14: 3188, 1975; Swales, Pharmac. Ther. 7: 173-201, 1979; Kokubu et al., Nature 217: 456-457, Feb. 3, 1968; Matsushita et al., J. Antibiotics 28: 1016-1018, Dec. 1975; Lazar et al., Biochem. Pharma. 23: 2776-2778, 1974; Miller et al., Biochem. Pharma. 21: 2941-2944, 1972; Haber, Clinical Science 59: 7s-19s, 1980; and Rich et al., J. Org. Chem. 43: 3624, 1978, and J. Med. Chem. 23: 27, 1980. Peptides which have the amino acid sequence Statyl-Ala-Statyl are known from the elder, non-prepublished EP-A 104 964. Those compounds are to serve, among other things, for treating high blood pressure.

Detailed description of the invention and preferred embodiments

In accordance with the present invention there are provided renin inhibitory peptides of the formula:

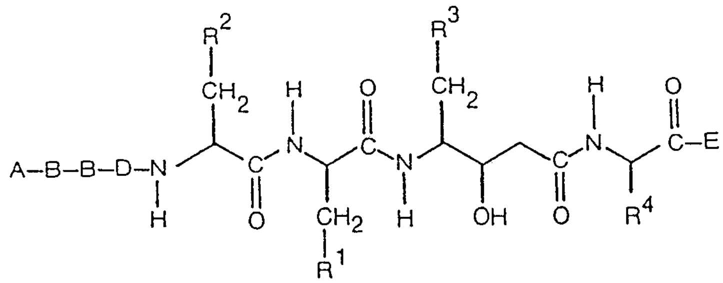

(I.)

wherein:

A is hydrogen;

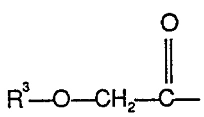; 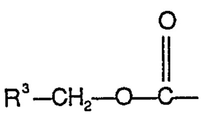;

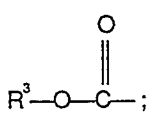; or 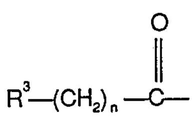

where n is 0 to 5 and $R^3$ has the same meaning as set out further below, and may additionally be hydrogen;

B is absent; glycyl; sarcosyl; or

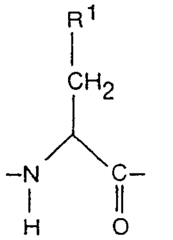;

D is absent; or

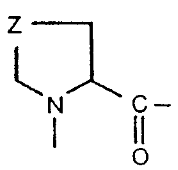

where Z is $(CH_2)_n$ and n is 1 or 2; or -S-;

$R^1$ is hydrogen; $C_{1-4}$ alkyl; hydroxy $C_{1-4}$ alkyl; phenyl; phenyl mono-substituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or $C_{3-7}$ cycloalkyl or phenyl mono-substituted with a member selected from the group consisting of methyl, trifluoromethyl, hydroxy, methoxy, fluoro, chloro, bromo, and iodo;

$R^4$ is hydrogen; or

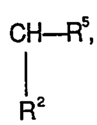

where $R^5$ is hydrogen; $C_{1-4}$ alkyl; hydroxy; or $C_{3-7}$ cycloalkyl; and

E is

(1) $-Y-(CH_2)_n-R^6$

where

Y is -NH- or -O-;

n is 0 to 5; and

$R^6$ is hydrogen; hydroxy; $C_{1-4}$ Alkyl; $C_{3-7}$ cycloal-

kyl; aryl; aryl substituted with up to five members independently selected from the group consisting of $C_{1-6}$ alkyl, trifluoromethyl, hydroxy, $C_{1-4}$ alkoxy, amino, mono- or di- $C_{1-4}$ alkylamino, and halo; amino; mono-, di-, or tri-$C_{1-4}$ alkylamino; guanidyl; heterocyclic; or heterocyclic substituted with up to five members independently selected from the group consisting of $C_{1-6}$ alkyl, hydroxy, trifluoromethyl, $C_{1-4}$ alkoxy, halo, aryl, aryl $C_{1-4}$ alkyl, amino, and mono- or di-$C_{1-4}$ alkylamino;

(2) 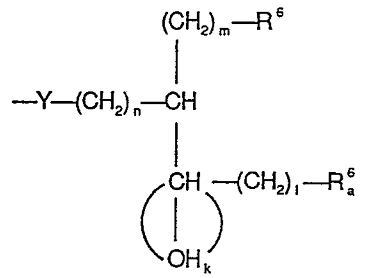

where
Y is as defined above;
n is 0 or 1;
k is 0 or 1;
l is 1 to 4;
m is 1 to 4; and
$R^6$ and $R_a^6$ may be the same or different and have the same meaning as $R^6$ above and $R_a^6$ may additionally be

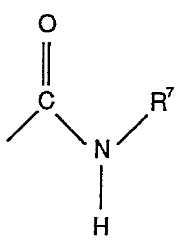 or 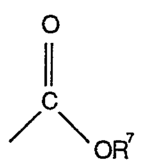

where $R^7$ is hydrogen or $C_{1-3}$ alkl; or

(3) 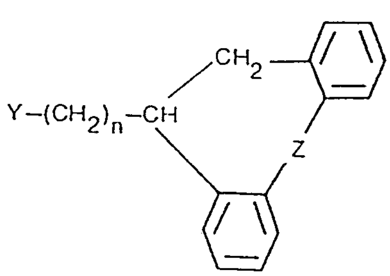

where
Y is as defined above; n is 0 or 1; and
Z is

(a) 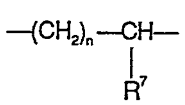

wherein
n is 0 or 1; and
$R^7$ is as defined above; or

(b) 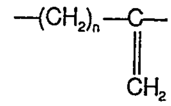

where
n is 0 or 1;
wherein all of the asymmetric carbon atoms have an S configuration, except for those in the B, D, and E substituents, which may have an S or R configuration; and a pharmaceutically acceptable salt thereof, with the exception of peptides of formula (I) and their salts in which
$R^3$ is 2-propyl,
$R^4$ is methyl,
E is 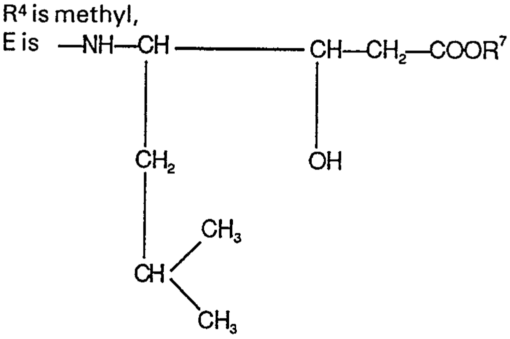

and B and D are absent.

While both the S and R chiralities for asymmetric carbon atoms in the E substituent are included in the peptides of the present invention, preferred chiralities are indicated in the description which follows.

In the above definitions, the term "alkyl" is intended to include both branched and straight chain hydrocarbon groups having the indicated number of carbon atoms.

The aryl substituent represents phenyl, naphthyl, or biphenyl.

The heterocyclic substituent recited above represents any 5- or 6-membered aromatic ring containing from one to three heteroatoms selected from the group consisting of nitrogen, oxygen, and sulfur; having various degrees of saturation; and including any bicyclic group in which any of the above heterocyclic rings is fused to a benzene ring. Heterocyclic substituents in which nitrogen is the heteroatom are preferred, and of these, those containing a single nitrogen atom are preferred. Fully saturated heterocyclic substituents are also preferred. Thus, piperidine is a preferred heterocyclic substituent. Other preferred heterocyclic substituents are: pyrryl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl and benzothienyl.

Where the heterocyclic substituent itself is substituted, it is preferred that the substituent be acyl$C_{1-4}$alkyl.

The novel renin inhibitory peptides of the present invention may also be described in terms of common amino acid components and closely related analogs thereof, in accordance with the following formula:

A—B—B—D—F—G—Sta—H—E (II.)

The A, B, D, and E components correspond to the same portions of Formula I.

In Formula II, Sta represents the unusual amino acid statine and its closely related analogs, and its presence constitutes a unique feature of the renin inhibitory peptides of the present invention. Statine may be named as 4(S)-amino-3(S)-hydroxy-6-methylheptanoic aid, and may be represented by the following formula:

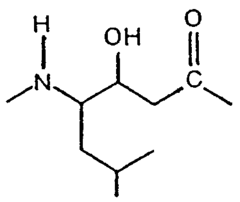
(III)

As shown in Formula III above, the delta-substituent in naturally-occurring statine is isopropyl, or a leucine sidechain, essentially. As shown in Formula I by the $R^3$ substituents, the isopropyl group may be replaced by higher alkyl groups up to six carbon atoms, cycloalkyl groups containing from three to seven carbon atoms, phenyl, and phenyl mono-substituted with a member selected from the group consisting of methyl, trifluoromethyl, hydroxy, methoxy, fluoro, chloro, bromo, and iodo. The phenyl substituent is especially preferred. These modifications of the naturally-occurring statine structure are in accordance with the hydrophobicity considered necessary to maintain the inhibitory activity of the total peptide.

The remaining common amino acid components of Formula II are as follows:

A has the same meaning as above in Formula I;
B is absent, Gly, Ala, Val, Leu, Ile, Phe, Tyr, Trp, His, Lys, Orn, Arg, or Met;
D is absent or Pro;
F is Ala, Leu, Phe, Tyr, or Trp;
G is Ala, Leu, Phe, Tyr, Trp, His, Lys, Orn, Arg, or Met;
H is the same as F and may additionally be Ser, Gly, Val, Ile, or Thr; and
E has the same meaning as above in Formula I.

It will be understood that closely related analogs of the above common amino acids, for example, aliphatic amino acids in addition to Ala, Val, Leu, and Ile, such as α-aminobutyric acid (Abu), and substituted phenyl derivatives of Phe, are included in the broad description of the novel inhibitory peptides of the present invention represented by Formula I and its definitions. Thus, the peptides of Formula II and its definitions, including the derivatives of naturally-occurring statine represented by the definitions of the $R^3$ substituent in Formula I, represent preferred peptides of the present invention.

Especially preferred inhibitory peptides of the present invention are the following:

IBU[1] - His-Pro-Phe-His-Sta-Leu-benzylamide;
IBU - His-Pro-Phe-His-Sta-Leu-2-phenylethylamide;
IBU - His-Pro-Phe-His-Sta-Leu-3-phenylpropylamide;
IBU - His-Pro-Phe-His-Sta-Leu-1,2-diphenylethylamide;
BOC[2] - Phe-His-Sta-Leu-(+)[3]-1,2-diphenylethylamide;
BOC - Phe-His-Sta-Leu-(-)-1,2-diphenylethylamide;
BOC - Phe-His-Sta-Leu-benzylamide;
BOC - Phe-His-Sta-Leu-(+)-α-phenylethylamide;
BOC - Phe-His-Sta-Leu-(-)-α-phenylethylamide;
BOC - Phe-His-Sta-Leu-(+)-α-naphthylethylamide;
BOC - Phe-His-Sta-Leu-(-)-α-naphthylethylamide;
BOC - Phe-His-Sta-Leu-p-chlorobenzylamide;
BOC - Phe-His-Sta-Leu-p-methoxybenzylamide;
BOC - Phe-His-Sta-Leu-10,11-dihydro-5H-dibenzo[a,d]-cyclohepteneamide;
BOC - Phe-His-Sta-Leu-D,L-threo-1,2-diphenyl-2-hydroxyethylamide;
BOC - Phe-His-Sta-Leu-Sta;
BOC - Phe-His-AHPPA[4]-Leu-benzylamide;
Acetyl - Phe-His-AHPPA-Leu-benzylamide;
BOC - Phe-His-Sta-Leu-(2-amidomethylpyridine);
BOC - Phe-His-Sta-Leu-(4-amidomethylpyridine);
BOC - Phe-His-Sta-Leu-(4-amido-1-benzylpiperidine);
BOC - Phe-His-Sta-Leu-[N-(3-amidopropyl)diethanolamine];
BOC - Phe-His-AHPPA-Leu-(2-amidomethylpyridine);
BOC - Phe-His-ACHPA[5]-Ile-(2-amidomethylpyridine);
IVA[6] - His-D-Pro-Phe-His-ACHPA-Ile-(2-amidomethylpyridine).

---

[1] IBU = Iso-butyryl.
[2] BOC = Tert-butyloxycarbonyl.
[3] (+) refers to the optical rotation of the amine.
[4] AHPPA = (3S, 4S)-4-amino-3-hydroxy-5-phenylpentanoic acid.
[5] ACHPA = (3S, 4S)-4-amino-5-cyclohexyl-3-hydroxypentanoic acid.
[6] IVA = Iso-valeryl.

The inhibitory peptides of the present invention may be better appreciated in terms of substrate analogy from the following illustration of Formula I alongside the octapeptide sequence of a portion of the pig renin substrate, which renin cleaves between $Leu^{10}$ and $Leu^{11}$.

| | His | Pro | Phe | His | Leu | Leu | Val | Tyr | |
|---|---|---|---|---|---|---|---|---|---|
| (5) | 6 | 7 | 8 | 9 | 10 | (11) | 12 | 13 | (14) |

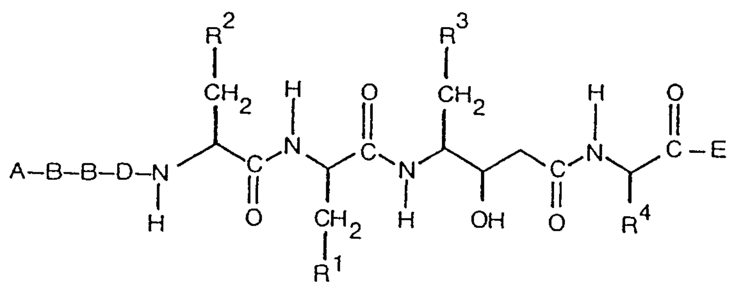

Statine

As can be seen a unique aspect and essential feature of the present invention is the substitution of the single statine anino acid component for the double amino acid sequence: $Leu^{10}$-$Leu^{11}$ in the endogenous pig renin substrate. It is believed that substitution of statine for both leucine amino acids rather than just one leucine results in an improved substrate analogy due to the greater linear extent of statine as compared to a single leucine component. Thus, statine more closely approximates Leu-Leu in linear extent, and thereby provides a better "fit" to the renin enzyme.

The inhibitory peptides of the present invention may also be better appreciated in terms of substrate analogy from the following illustration of Formula I alongside the octapeptide sequence of a portion of the human renin substrate, which renin cleaves between $Leu^{10}$ and $Val^{11}$:

| | His | Pro | Phe | His | Leu | Leu | Val | Tyr | |
|---|---|---|---|---|---|---|---|---|---|
| (5) | 6 | 7 | 8 | 9 | 10 | (11) | 12 | 13 | (14) |

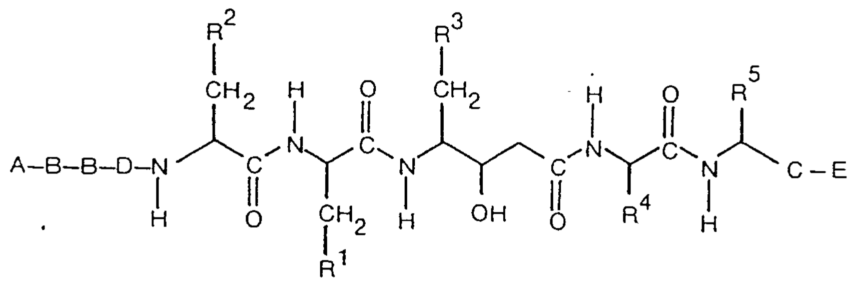

Statine

As can be seen, a unique aspect and essential feature of the present invention is the substitution of the single statine amino acid component for the double amino acid sequence: $Leu^{10}$-$Val^{11}$ in the endogenous human renin substrate. It is believed that substitution of statine for both the leucine and valine amino acids rather than just the leucine results in an improved substrate analogy due to the greater linear extent of statine as compared to a single leucine component. Thus, statine more closely approximates Leu-Val in linear extent, and thereby provides a better "fit" to the human renin enzyme.

In the endogenous substrate it is also preferred to substitute Leu for $Val^{12}$ and Phe for $Tyr^{13}$ in order to enhance the inhibitory activity of the resulting peptide.

The Formula I compounds can be used in the form of salts derived from inorganic or organic acids and bases. Included among such acid addition salts are the following: acetate, adipate, alginate, aspartate, benzoate, benzenesulfonate, bisulfate, butyrate citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

The peptides of the present invention possess an excellent degree of activity in treating renin-associated hypertension and hyperaldosteronism.

For these purposes the compounds of the present invention may be administered parenterally, by inhalation spray, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The peptides of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Dosage levels of the order of 2 to 35 grams per day are useful in the treatment of the above indicated conditions. For example, renin-associated hypertension and hyperaldosteronism are effectively treated by the administration of from 30 milligrams to 0.5 grams of the compound per kilogram of body weight per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Thus, in accordance with the present invention there is further provided a pharmaceutical composition for treating renin-associated hypertension and hyperaldosteronism, comprising a pharmaceutical carrier and a therapeutically effective amount of a peptide of the formula:

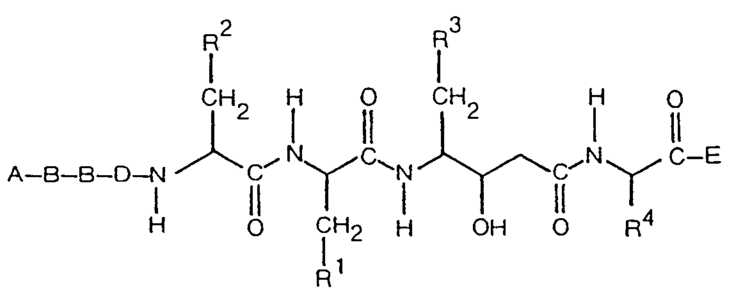

(I.)

wherein A, B, D, $R^1$, $R^2$, $R^3$, $R^4$, and E have the same meaning as recited further above for Formula 1; wherein all of the asymmetric carbon atoms have an S configuration, except for those in the B, D, and E substituents, which may have an S or R configuration; or of a pharmaceutically acceptable salt thereof.

Also, in accordance with the present invention

there is still further provided a method of treating renin-associated hypertension and hyperaldosteronism, comprising administering to a patient in need of such treatment, a therapeutically effective amount of a peptide of the formula:

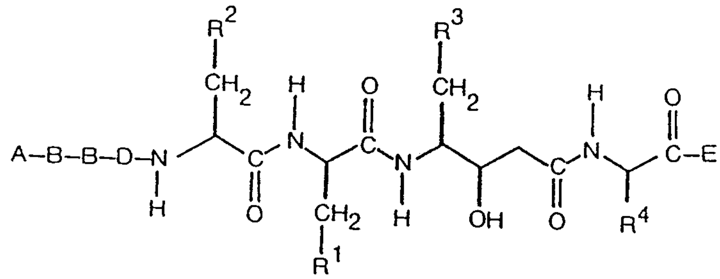

(I.)

wherein A, B, D, $R^1$, $R^2$, $R^3$, $R^4$, and E have the same meaning as recited further above for Formula I; wherein all of the asymmetric carbon atoms have an S configuration, except for those in the B, D, and E substituents, which may have an S or R configuration; or of a pharmaceutically acceptable salt thereof.

The renin inhibitory peptides of the present invention may also be utilized in diagnostic methods for the purpose of establishing the significance of renin as a causative or contributory factor in hypertension or hyperaldosteronism in a particular patient. For this purpose the peptides of the present invention may be administered in a single dose of from 0.1 to 10 mg per kg of body weight.

Both in vivo and in vitro methods may be employed. In the in vivo method, a peptide of the present invention is administered to a patient, preferably by intravenous injection, although parenteral administration is also suitable, at a hypotensive dosage level and as a single dose, and there may result a transitory fall in blood pressure. This fall in blood pressure, if it occurs, indicates supranormal plasma renin levels.

An in vitro method which may be employed involves incubating a body fluid, preferably plasma, with a novel peptide of the present invention and, after deproteinization, measuring the amount of angiotensin II produced in nephrectomized, pentolinium-treated rats. Another in vitro method involves mixing the plasma or other body fluid with a novel peptide of the present invention and injecting the mixture into a test animal. The difference in a pressor response with and without added peptide is a measure of the renin content of the plasma.

Pepstatin may be employed in the methods described above as an active control. See, e.g., U.S. Patent Nos. 3 784 686 and 3 873 681 for a description of the use of pepstatin in diagnostic methods of this type.

The peptides of the present invention may be prepared in accordance with well-known procedures for preparing peptides from their constituent amino acids, which will be described in more detail below. The unusual amino acid, statine, may be prepared in accordance with the procedure described by Rich et al., J. Org. Chem. 43: 3624 (1978).

A general method of preparation may be described in the following terms:

A method of preparing a peptide of formula I, said peptide being comprised of from five to seven amino acids identified as I through VII, amino acid (AA) I being at the C-terminus of said peptide, to which substituent E is attached, and amino acid (AA) VII being at the N-terminus of said peptide, to which substituents A-B-B-D are attached, comprising the steps of:

(A) treating the desired ester or amide of the C-terminus amino acid (AA I) with the next adjacent amino acid (AA II) of said peptide, the amino group of said amino acid being protected by a protecting group, in the presence of a condensing agent, whereby a dipeptide of the two amino acids (AA I and II) is formed;

(B) deprotecting the dipeptide formed in Step (A) by removing the protecting group from the amino group of AA II;

(C) treating the dipeptide of AA I and AA II with AA III, the amino group of which is protected by a protecting group, in the presence of a condensing agent, whereby a tripeptide of AA I, AA II and AA III is formed;

(D) deprotecting the tripeptide formed in Step (C) by removing the protecting group from the amino group of AA III;

(E) forming a quadripeptide up to a heptapeptide of AA I, through AA IV, AA V, AA VI or AA VII, by repeating the procedure of Step (C) using protected AA IV through protected AA VII;

(F) deprotecting the quadripeptide through heptapeptide formed in Step (E) to give the peptide of formula I wherein A is hydrogen; and optionally

(G) treating the quadripeptide through heptapeptide formed in Step (F) with

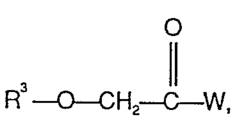, 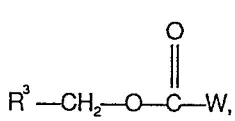, 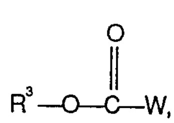, or 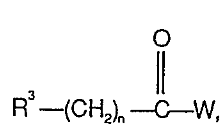,

where $R^3$ and n are as defined above and W is an acid halide, anhydride, or other carboxyl activating group, to give the peptide of formula I wherein A is other than hydrogen and optionally
(H) treating the quadripeptide through heptapeptide formed in Steps (F) or (G) where an ester of AA I is employed with hydrazine to give the corresponding hydrazide, followed by treatment of said hydrazide with acidic nitrite to give the corresponding acyl azide, followed by treatment of said acyl azide with the appropriate amine compound to give the desired E substituent in the peptide of Formula I;
said method also comprising, where necessary, protection of sidechain substituents of the component amino acids AA I through AA VII, with deprotection being carried out as a final step; said method also comprising any combination of the steps set out above, whereby the amino acids I through VII and substituents A, B, D, and E are assembled in any desired order to prepare the peptide of formula I; and said method also comprising employment of the steps set out above in a solid phase sequential synthesis, whereby in the initial step the carboxyl group of the selected amino acid is bound to a synthetic resin substrate while the amino group of said amino acid is protected, followed by removal of the protecting group, the succeeding steps being as set out above, the peptide as it is assembled being attached to said synthetic resin substrate; followed by a step of removing the peptide of formula I from said synthetic resin substrate: (a) by strong acid cleavage to give E = OH; (b) by transesterification with a $C_{1-4}$ alkanol to give E = O-$C_{1-4}$ alkyl (followed by hydrolysis to give E = OH); or (c) by ammonolysis with $NH_2R'$ where R' is hydrogen or $C_{1-4}$ alkyl; and after removal of the peptide of Formula I from said synthetic resin substrate by transesterification to form the ester thereof as recited above, optionally the step of teating said ester thereof in accordance with the procedures described in Step (H) above to give the desired E substituent in the peptide of Formula I; removal of sidechain protecting groups being accomplished either before or after removal of the peptide of Formula I from said synthetic resin substrate.

Preparation of the peptides of Formula I having the desired E substituent, as described above in Step (H), may be illustrated as follows for the particular case where E = benzylamide (and PEP represents the remaining portion of the peptide of Formula I):

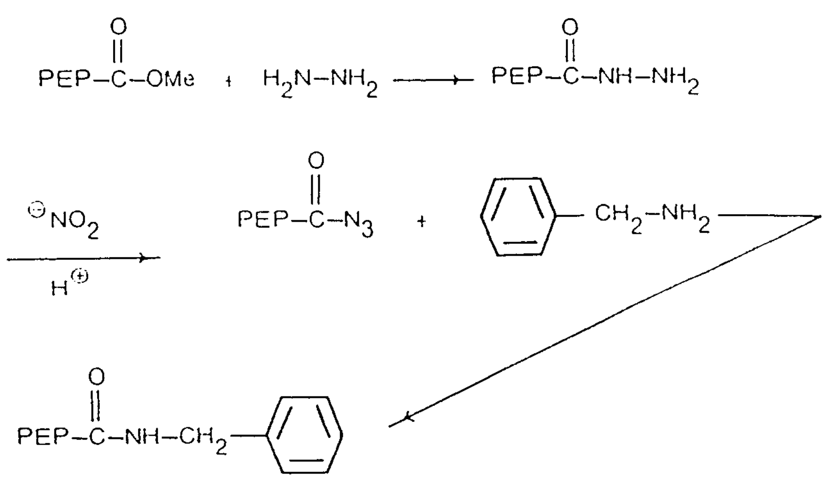

The phenyl analog of statine, (3S, 4S)-4-amino-3-hydroxy-5-phenylpentanoic acid (AHPPA) can be prepared in accordance with the procedure described by Rich et al., J. Med. Chem. 23: 27-33 (1980).

The cyclohexylalanine analog of statine, (3S, 4S)-4-amino-5-cyclohexyl-3-hydroxypentanoic acid (ACHPA) can be prepared by catalytic hydrogenation (using $H_2$/Rh on alumina, or other suitable catalyst) of the BOC-AHPPA, prepared as described in the paragraph immediately above. Alternatively, this and similar statine analogs can be prepared in accordance with the procedure desdribed for statine, where the BOC-Leu starting material is replaced with the amino acid containing the desired side chain. Thus, BOC-ACHPA can also be prepared starting from BOC-L-cyclohexylalanine, itself prepared, for example, by catalytic reduction of BOC-Phe, in the same manner as described for BOC-AHPPA.

The novel inhibitory peptides of the present invention are prepared by using the solid phase sequential synthesis technique.

In the following description several abbreviated designations are used for the amino acid components, certain preferred protecting groups reagents and solvents. The meanings of such abbreviated designations are given below in Table I.

Table I

| Abbreviated Designation | Amino Acid |
|---|---|
| AHPPA | (3S,4S)-4-amino-3-hydroxy-5-phenylpentanoic acid |
| ACHPA | (3S,4S)-4-amino-5-cyclohexyl-3-hydroxypentanoic acid |
| Ala | L-alanine |
| Arg | L-arginine |
| Gly | L-glycine |
| His | D or L-histidine |
| Ile | L-isoleucine |
| Leu | L-leucine |
| Lys | L-lysine |
| Met | L-methionine |
| Orn | L-ornithine |
| Phe | L-phenylalanine |
| Pro | D or L-proline |
| Ser | L-serine |
| Sar | L-sarcosine (N-methylglycine) |
| Sta | (3S,4S)-statine |
| Thr | L-threonine |
| Trp | L-tryptophan |
| Tyr | L-tyrosine |
| Val | L-valine |

| Abbreviated Designation | Protecting Groups |
|---|---|
| BOC | tert-butyloxycarbonyl |
| CBZ | benzyloxycarbonyl |
| DNP | dinitrophenyl |
| OMe | methyl ester |

| Abbreviated Designation | Activating Groups |
|---|---|
| HBT | 1-hydroxybenzotriazole |

| Abreviated Designation | Condensing Agents |
|---|---|
| DCCI | dicyclohexylcarbodiimide |
| DPPA | diphenylphosphorylazide |

| Abbreviated Designation | Reagents |
|---|---|
| TEA | triethylamine |
| TFA | trifluoroacetic acid |

| Abbreviated Designation | Solvents |
|---|---|
| A | ammonium hydroxide (conc.) |
| AcOH | actic acid |
| C | chloroform |
| DMF | dimethyl-formamide |
| E | ethyl acetate |
| M | methanol |
| P | pyridine |
| THF | tetrahydrofuran |
| W | water |

The synthesis of the peptides of the present invention by the solid phase technique is conducted in a stepwise manner on chloromethylated resin. The resin is composed of fine beads (20–70 microns in diameter) of a synthetic resin prepared by copolymerization of styrene with 1–2 percent divinylbenzene. The benzene rings in the resin are chloromethylated in a Friedel-Crafts reaction with chloromethyl methyl ether and stannic chloride. The Friedel-Crafts reaction is continued until the resin contains 0.5 to 5 mmoles of chlorine per gram of resin.

The amino acid selected to be the C-terminal amino acid of the linear peptide is converted to its amino protected derivative. The carboxyl group of the selected C-terminal amino acid is bound covalently to the insoluble polymeric resin support, as for example, as the carboxylic ester of the resin-bonded benzyl chloride present in chloromethyl-substituted polystyrene-divinylbenzene resin. After the amino protecting group is removed, the amino protected derivative of the next amino acid in the sequence is added along with a coupling agent, such as dicyclohexylcarbodiimide. The amino acid reactant may be employed in the form of a carboxyl-activated amino acid such as ONP ester an amino acid azide, and the like. Deprotection and addition of successive amino acids is performed until the desired linear peptide is formed.

The selection of protecting groups is, in part, dictated by particular coupling conditions, in part by the amino acid and peptide components involved in the reaction.

Amino-protecting groups ordinarily employed include those which are well known in the art, for example, urethane protecting substituents such as benzyloxy-carbonyl (carbobenzoxy), p-methoxycarbobenzoxy, p-nitrocarbobenzoxy, t-butoxycarbonyl, and the like. It is preferred to utilize t-butyloxycarbonyl (BOC) for protecting the α-amino group in the amino acids undergoing reaction at the carboxyl end of said amino acid. The BOC protecting group is readily removed following such coupling reaction and prior to the subsequent step by the relatively mild action of acids (i.e. trifluoroacetic acid, or hydrogen chloride in ethyl acetate).

The OH group of Thr and Ser can be protected by the Bzl group and the -amino group of Lys can be protected by the INOC group or the 2-chlorobenzyloxycarbonyl (2-Cl-CBZ) group. Neither group is affected by TFA, used for removing BOC protecting groups.

After the peptide is formed, the protective groups, such as 2-Cl-CBZ and Bzl, can be removed by treatment with HF or by catalytic hydrogenation.

After the peptide has been formed on the solid phase resin, it may be removed from the resin by a variety of methods which are well known in the art. For example the peptide may be cleaved from the resin with hydrazine, by ammonia in methanol, or by methanol plus a suitable base.

Preparation of the novel inhibitory peptides of the present invention utilizing the solid phase technique is illustrated in the following examples. These examples have actually been carried out. However, they are not intended to be any limitation of the present invention.

Example 1
N-isobutyryl-L-histidyl-L-prolyl-L-phenylalanyl-L-histidyl-(3s,4S)-statyl-L-leucyl-benzylamide

The title peptide was prepared by standard solid phase methodology, as described in Erickson and Merrifield, Proteins, 3rd et., 2: 257–527, 1976, using a Beckman Model 990B peptide synthesizer to carry out the operations according to the attached programs.

A. Isobutyryl-L-histidyl-L-prolyl-L-phenylalanyl-L-histidyl-(3S,4S)-statyl-L-leucyl-O-Resin

The starting polymer was BOC-Leu esterified to 2% cross-linked polystyrene-divinylbenzene (6 mmol, 5.00 g). The $N^{\alpha}$-BOC derivatives of Sta, His-DNP, Phe, and Pro were coupled using dicyclohexylcarbodiimide with an equivalent of the additive 1-hydroxybenzotriazole hydrate. The Sta was prepared in accordance with Rich et al., J. Org. Chem. 43: 3624, 1978. The BOC-group was removed with 40% trifluoroacetic acid. A coupling of 60 minutes followed by a recoupling of 120 minutes (2.5 equivalents each time of BOC-amino acid) were used for each amino acid, except for Sta. These coupling times had been previously demonstrated to give complete coupling (as judged by the method of Kaiser) in this sequence. In order to conserve the amounts of Sta employed, an initial coupling using 1.08 equivalents of $N^{\alpha}$-BOC-Sta (in 20 ml of 1:1 $CH_2Cl_2$/DMF) for 72 hrs gave approximately 95% complete reaction. The addition of an additional 0.12 equivalents of $N^{\alpha}$-BOC-Sta plus equal amount of DCCI to the stirring suspension gave complete coupling after an additional 18 hrs. The N-terminal isobutyryl group was coupled for 60 minutes as the symmetrical anhydride generated in situ from 5.0 equivalents of isobutyric acid and 2.5 equivalents of DCCI. This was followed by a recoupling for 120 minutes using 2.5 equivalents of isobutyric acid, HBT, and DCCI. The DNP protecting groups on His were removed in the final program using two 25-minute treatments with 10% thiophenol in DMF. The finished resin-peptide (2.70 g) was dried and suspended in 40 ml of dry methanol.

Schedule of steps for 6 mmol. run

| Step | Solvent/Reagent | Vol. (ml) | Mix time (min) |
|---|---|---|---|
| Coupling Program 1 | | | |
| 1 | $CH_2Cl_2$ | 6 × 60 | 2 |
| 2 | 40% TFA in $CH_2Cl_2$ | 1 × 60 | 2 |
| 3 | 40% TFA in $CH_2Cl_2$ | 1 × 60 | 25 |
| 4 | $CH_2Cl_2$ | 3 × 60 | 2 |
| 5 | 10% TEA in $CH_2Cl_2$ | 2 × 60 | 5 |
| 6 | $CH_2Cl_2$ | 3 × 60 | 2 |
| 7 | BOC-amino acid, HBT in 1:1 DMF/$CH_2Cl_2$ | 40 | 5 |
| 8 | 1.OM DCCI in $CH_2Cl_2$ | 15 | 60 |
| 9 | DMF | 1 × 60 | 2 |
| 10 | MeOH | 2 × 60 | 2 |
| 11 | $CH_2Cl_2$ | 1 × 60 | 2 |
| Re-Couple Program 2 | | | |
| 1 | $CH_2Cl_2$ | 1 × 60 | 2 |
| 2 | 10% TEA in $CH_2Cl_2$ | 2 × 60 | 5 |
| 3 | $CH_2Cl_2$ | 3 × 60 | 2 |
| 4 | BOC-amino acid, HB in 1:1 DMF/$CH_2Cl_2$ | 40 | 5 |
| 5 | 1.OM DCCI in $CH_2Cl_2$ | 15 | 120 |
| 6 | DMF | 1 × 60 | 2 |
| 7 | MeOH | 2 × 60 | 2 |
| 8 | $CH_2Cl_2$ | 5 × 60 | 2 |
| Program 3 (DNP remova l) | | | |
| 1 | $CH_2Cl_2$ | 1 × 60 | 2 |
| 2 | DMF | 2 × 60 | 2 |

Schedule of steps for 6 mmol run

| Step | Solvent/Reagent | Vol. (ml) | Mix time (min) |
|---|---|---|---|
| 3 | 10% phenylthiol in DMF | 1 × 60 | 25 |
| 4 | DMF | 1 × 60 | 2 |
| 5 | 10% TEA in $CH_2Cl_2$ | 1 × 60 | 2 |
| 6 | DMF | 2 × 60 | 2 |
| 7 | 10% phenylthiol in DMF | 1 × 60 | 25 |
| 8 | DMF | 3 × 60 | 2 |
| 9 | MeOH | 2 × 60 | 2 |
| 10 | $CH_2Cl_2$ | 2 × 60 | 2 |
| 11 | MeOH | 2 × 60 | 2 |
| 12 | $CH_2Cl_2$ | 2 × 60 | 2 |
| 13 | MeOH | 2 × 60 | 2 |

B. Isobutyryl-L-histidyl-L-prolyl-L-phenylalanyl-L-histidyl-(3S,4S)-statyl-L-leucine methyl ester

To the suspension prepared in Step A above was added 10 ml diisopropylethylamine, and the reaction mixture was stirred under a dry nitrogen atmosphere for 18 hours. The reaction mixture was then filtered and the yellow solution was evaporated under reduced pressure to give 1.4 g of crude methyl ester. This crude product was dissolved in 50 ml of methylene chloride and washed with water. The methylene chloride layer was dried over sodium sulfate and evaporated to give 1.1 g of yellow powder. This material was chromatographed on a silica column (160 g, 0.04–0.063 mmol) packed and eluted ith chloroform/methanol/water/acetic acid – 120:20:1.6: 0.4. The pure methyl ester (thin layer chromatography on silica – chloroform/methanol/water – 80:20:2, Rf = 0.43) was obtained by evaporation of the appropriate fractions and precipitation from 3 ml of methylene chloride/50 ml of petroleum ether. Yield was 0.74 g.

C. Isobutyryl-L-hystidyl-l-prolyl-L-phenylalanyl-L-histidyl-(3S,4S)-statyl-L-leucine hydrazide

A portion (0.44 g) of the ester prepared in Step B above was converted to the hydrazide by dissolving it in 2 ml of a 1:1 mixture of dry methanol and anhydrous hydrazine. After a few minutes the solution was evaporated to dryness, at 30°C, and the crude hydrazide was dissolved in 15 ml of n-butanol and washed 5 times with an equal volume of water containing a small amount of sodium chloride. The n-butanol layer was then evaporated and the hydrazide was precipitated from methylene chloride/petroleum ether. The yield was 0.41 g of a single material; thin layer chromatography (silica) 80:10:1 – chloroform/methanol/conc. ammonia, Rf = 0.12 (compare Rf = 0.19 for the methyl ester). The material was over 99% pure as determined by high performance liquid chromatography.

D. N-Isobutyryl-L-histidyl-L-prolyl-L-phenylalanyl-L-histidyl-(3S,4S)-statyl-L-leucylbenzylamide

The hydrazide prepared in Step C above can be converted by acidic nitrite to its corresponding acyl azide, and coupled to a wide variety of amines. Thus, 0.10 g of the hydrazide (0.10 mmol) was dissolved in 0.5 ml of dry, degassed dimethylformamide, and cooled to –30°C under a nitrogen atmosphere. To this cold solution was added 1.1 mmol of fresh 7.1 N hydrochloric acid in dry tetrahydrofuran. To this acidic solution was then added 15 µl of isoamylnitrite over a period of 10 minutes. After approximately 1 hour, there was added 1.1 mmol of diisopropylethylamine, and the pH was adjusted with additional base to 7 (using pH 6–8 paper). To this solution of the acyl azide was added 2 equivalents (0.2 mmol) of benzylamine, and the reaction mixture was held at –20°C for 18 hours. The reaction solution was evaporated, and the resulting yellow oil was triturated with ether. The remaining solid was dissolved in 10 ml of n-butanol and washed twice with water, once with 5% sodium bicarbonate, and twice with sodium chloride solution. The butanol layer was then evaporated, dissolved in 10 ml methylene chloride, and the product was precipitated with 50 ml of ether. The yield was 60 mg showing a single spot on thin layer chromatography 80:20:2 – chloroform/methanol/conc. ammonia, Rf = 0.47. The product was 93.1% pure, as determined by high performance liquid chromatography, and had an acceptable amino acid analysis: His 1.98, pro 0.96, Phe 1.03, and Leu 1.01. A 300 MHz 'HNMR spectrum was consistent with the desired structure.

Example 2–26

Following the standard solid phase methodology described above in Example 1, additoinal inhibitory peptides of the present invention were prepared. The peptides prepared are set out in the following table, wherein the subscribed numerical value for each amino acid indicates the results of the Spinco amino acid analysis.

| Exm. No. | Peptide |
|---|---|
| 2 | IBU - His - Pro - Phe - His - Sta - Leu - NH - $(CH_2)_2$ 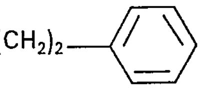<br>1.01 1.00 0.98 1.01 1.00<br>[-2-phenylethylamide] |
| 3 | IBU- His- Pro- Phe- His- Sta Leu- NH- $(CH_2)_3$ 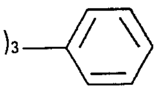<br>0.99 1.00 1.01 0.99 1.01<br>[-3-phenylpropylamide] |
| 4 | IBU- His- Pro- Phe- His- Sta- Leu- NH- $(CH_2)_4$ 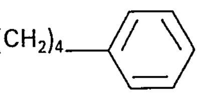<br>1.02 0.97 1.01 1.02 0.99<br>[-4-phenylbuthylamide] |
| 5 | IBU- His- Pro- Phe- His- Sta- Leu- NH 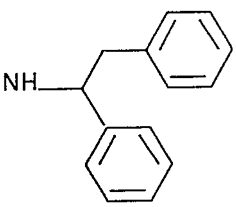<br>1.01 1.04 0.97 1.01 0.98<br>[-1,2-diphenylethylamide] |
| 6 | BOC-Phe-His-Sta-Leu-$OCH_3$<br>[tert-Butyloxycarbonyl-L-phenyl la lanyl-L-histidyl-(3S,4S)-statyl-L-leucine methyl ester] |
| 7 | BOC - Phe-His-Sta-Leu-NH 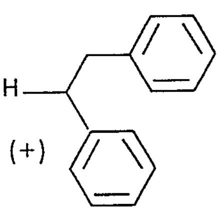<br>1.00 1.00 1.00 (+)<br>[-L-leucyl-(+)-1,2-diphenylethylamide] |
| 8 | BOC-Phe-His-Sta-Leu-NH 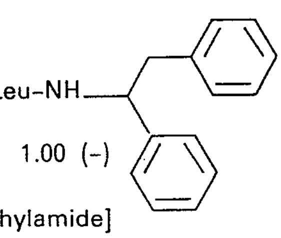<br>1.00 1.00 1.00 (-)<br>[-(-)-1,2-diphenylethylamide] |

| Exm. No. | Peptide |
|---|---|
| 9 | BOC-Phe-His-Sta-Leu-NH-$CH_2$ 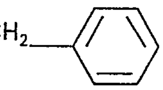<br>1.00 1.10 1.00<br>[benzylamide] |
| 10 | BOC-Phe-His-Sta-Leu-NH 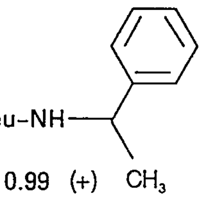<br>1.03 0.98 0.99 (+)<br>[-(+)-α-phenylethylamide] |
| 11 | BOC-Phe-His-Sta-Leu-NH 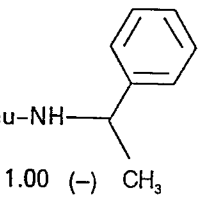<br>1.00 1.00 1.00 (–)<br>[-(-)-α-phenylethylamide] |
| 12 | BOC-Phe-His-Sta-Leu-NH 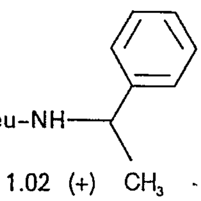<br>1.01 0.98 1.02 (+)<br>[-(+)-α-naphthylethylamide] |
| 13 | BOC-Phe-His-Sta-Leu-NH 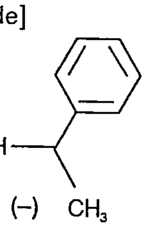<br>1.02 0.97 1.00 (–)<br>[-(-)-α-naphthylethylamide] |
| 14 | BOC-Phe-His-Sta-Leu-NH- $CH_2$ 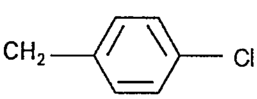<br>1.00 0.99 1.01<br>[-p-chlorobenzylamide] |
| 15 | BOC-Phe-His-Sta-Leu-NH- $CH_2$ 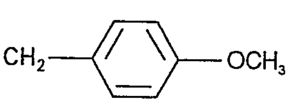<br>0.99 1.00 1.01<br>[-p-methoxybenzylamide] |

| Exm. No. | Peptide |
|---|---|
| 16 | BOC-Phe-His-Sta-Leu-NH 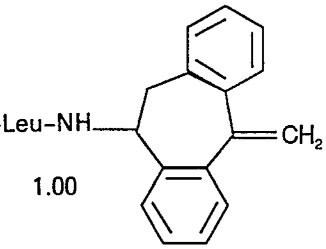<br>1.00 1.00 1.00<br>[-10,11-dihydro-5H-dibenzo[a,d]-cycloheptene amide] |
| 17 | BOC-Phe-His-Sta-Leu-NH 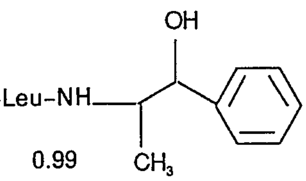<br>1.01 1.01 0.99<br>[-norpseudoephedrylamide] |
| 18 | BOC-Phe-His-Sta-Leu-NH 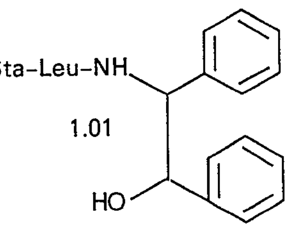<br>0.99 1.00 1.01<br>[-D,L-erythro-1,2-diphenyl-2-hydroxyethylamide] |
| 19 | BOC-Phe-His-Sta-Leu-NH 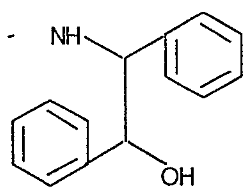<br>1.00 0.99 1.02<br>[-D,L-threo-1,2-diphenyl-2-hydroxyethylamide] |
| 20 | BOC-Phe-His-Sta-Leu-NH-$CH_2$ 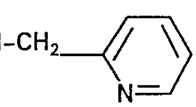<br>[-2-amidomethylpyridine] |
| 21 | BOC-Phe-His-Sta-Leu-NH-$CH_2$ 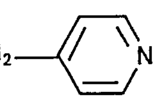<br>[-4-amidomethylpyridine] |
| 22 | BOC-Phe-His-Sta-Leu-NH 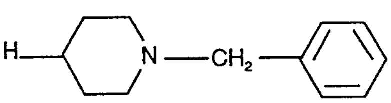<br>1.02 1.00 0.98<br>[-4-amido-1-benzylpiperidine] |

| Exm. No. | Peptide |
|---|---|
| 23 | BOC-Phe-His-Sta-Leu-NH-$(CH_2)_3$-N-$(CH_2CH_2OH)_2$<br>1.02 1.01 0.98<br>[-N-(3-amidopropyl)-diethanolamine] |
| 24 | Acetyl- Phe -His -AHPPA -Leu -NH - $CH_2$ 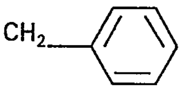<br>1.01 1.00 0.99 |
| 25 | BOC-Phe-His-AHPPA-Leu-NH-$CH_2$ 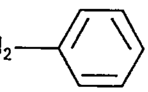<br>1.00 0.99 1.01 |
| 26 | BOC-Phe-His-Sta-Leu-Sta<br>0.99 1.00 1.01 |

For the peptides prepared above, various analytical methods were carried out to verify the structure of the peptides products. The following table indicates which methods were employed and summarizes the results where practicable.

Analytical Method

| Example No. | TLC[1] (No. of systems) | HPLC[2] | AA[3] | NMR[4] |
|---|---|---|---|---|
| 2 | 97% (2) | 97.1% | X | X |
| 3 | 97% (2) | 92.7% | X | X |
| 4 | 97% (2) | 93.8% | X | X |
| 5 | 98% (4) | 99% | X | X |
| 6 | 98% (2) | 99% | — | X |
| 7 | 98% (1) | 94.0% | X | X |
| 8 | 98% (1) | 99% | X | X |
| 9 | 98% (1) | 97.8% | X | X |
| 10 | 98% (1) | 99.5% | X | X |
| 11 | 98% (1) | 99% | X | X |
| 12 | 98% (1) | 93% | X | X |
| 13 | 98% (1) | 85% | X | X |
| 14 | 98% (1) | | X | X |
| 15 | 98% (1) | 95.5% | X | X |
| 16 | 98% (1) | 96.7% | — | X |
| 17 | 98% (1) | 98.7% | X | X |
| 18 | 98% (1) | 98.5% | X | X |
| 19 | 98% (1) | 91% | X | X |
| 20 | 98% (1) | — | — | X |
| 21 | 98% (1) | — | — | X |

Analytical Method

| Example No. | TLC[1] | HPLC[2] | AA[3] | NMR[4] |
|---|---|---|---|---|
| 22 | 98% (1) | 90.7% | X | X |
| 23 | 98% (1) | 98.3% | X | X |
| 24 | 95% (1) | 92.1% | — | X |
| 25 | 95% (1) | 95.9% | X | X |
| 26 | 95% (1) | 95.0% | — | X |

[1]TLC = thin layer chromatography on silica gel; visualization by reagents which tend to detect peptides; no. of systems refers to number of different solvent mixtures used to elute chromatograms; % refers to estimated purity.
[2]HPLC = high pressure liquid chromatography; detection by ultraviolet absorption at 240 nm or 210 nm; chromatography is reverse phase, values should be 1.00 ± 0.03
[3]AA = amino acid analysis; peptides are hydrolyzed to their component amino acids, which are then quantitatively measured; values should be 1.00 ± 0.03.
[4]NMR = nuclear magnetic resonance spectroscopy at 300 MHz or 360 MHz for protons; X = spectrum consistent with structure; — = not performed.

Example 27
Hog Renin Inhibition

An assay was carried out in order to determine the inhibitory potency of the peptides of the present invention. The assay measured the inhibition of hog kidney renin, and was in accordance with the procedure described in Rich et al., J. Med. Chem. 23: 27, 1980, except that a pH of 7.3 was used. The results of the assay, illustrated in the table below, are expressed as $I_{50}$ values, which refers to the concentration of peptide inhibitor necessary to produce 50% inhibition of renin activity. This $I_{50}$ value is obtained typically by plotting data from four inhibitor concentrations. Pepstatin was used as an active control.

| Peptide | $I_{50}$(M) |
|---|---|
| IBU[1]– His-Pro-Phe-His-Sta-Leu-benzylamide; | $1.3. \times 10^{-8}$ |
| IBU – His-Pro-Phe-His-Sta-Leu-2-phenylethylamide; | $3.0 \times 10^{-8}$ |
| IBU – His-Pro-Phe-His-Sta-Leu-3-phenylpropylamide; | $1.9 \times 10^{-8}$ |
| IBU – His-Pro-Phe-His-Sta-Leu-1,2-diphenylethylamide; | $5.6 \times 10^{-9}$ |
| BOC[2]– Phe-His-Sta-Leu-(+)[3]-1,2-diphenylethylamide; | $6.7 \times 10^{-9}$ |
| BOC – Phe-His-Sta-Leu-(−) -1,2-diphenylethylamide; | $2.3 \times 10^{-8}$ |
| BOC – Phe-His-Sta-Leu-benzylamide; | $1.1 \times 10^{-8}$ |
| BOC – Phe-His-Sta-Leu-(+)-α-phenylethylamide; | $1.4 \times 10^{-8}$ |
| BOC – Phe-His-Sta-Leu-(−)-α-phenylethylamide; | $2.2 \times 10^{-8}$ |
| BOC – Phe-His-Sta-Leu-(+)-α-naphtylethylamide; | $1.1 \times 10^{-8}$ |
| BOC – Phe-His-Sta-Leu-(−)-α-naphthylethylamide; | $1.4 \times 10^{-8}$ |
| BOC – Phe-His-Sta-Leu-p-chlorobenzylamide; | $5.0 \times 10^{-9}$ |
| BOC – Phe-His-Sta-Leu-p-methoxybenzylamide; | $6.7 \times 10^{-9}$ |
| BOC – Phe-His-Sta-Leu-10,11-dihydro-5H-dibenzo[a,d]-cyclohepteneamide; | $5.8 \times 10^{-9}$ |
| BOC – Phe-His-Sta-Leu-D,L-threo-1,2-diphenyl-2-hydroxyethylamide; | $2.4 \times 10^{-8}$ |
| Acethyl – Phe-His-AHPPA-Leu-benzylamide; | $3.4 \times 10^{-9}$ |
| BOC – Phe-His-AHPPA-Leu-benzylamide; | $1.4 \times 10^{-9}$ |
| BOC – Phe-His-Sta-Leu-Sta. | $3.6 \times 10^{-8}$ |

[1] IBU = Iso-butyryl.
[2] BOC = Tert-butyloxycarbonyl.
[3] (+) refers to the optical rotation of the amine.

Example 28
Human Renin Inhibition

An assay was carried out in order to determine the inhibitory potency of the peptides of the present invention. The assay measured the inhibition of human kidney renin purified as described in Bangham, D.R., Robertson, I., Robinson, J.I.S., Robinson, C.J., and Tree, M., Clinical Science and Molecular Medicine, 48 (Supp. 2): 136s–159s (1975), and further purified by affinity chromatography on pepstatin-aminohexyl-Sepharase as described in Poe, M., Wu., J.K., Florance, J.R., Radkey, J.A., Bennett, C.D., and Hoagsteen, K., J. Biol. Chem. (1982, in press). The assay was also in accordance with Poe et al. cited above. Results are expressed as $K_I$ values, which refer to the dissociation constant of the inhibited enzyme – inhibitor complex. This $K_I$ value was obtained as described above. Pepstatin was used as an active control. The results are set out in the table below.

| Peptide | $K_i(M)$ |
|---|---|
| IBU – His-Pro-Phe-His-Sta-Leu-benzylamide; | $1.2 \times 10^{-8}$ |
| BOC – Phe-His-Sta-Leu-(+)-1,2-diphenylethylamide; | $2.9 \times 10^{-8}$ |
| BOC – Phe-His-Sta-Leu-benzylamide; | $1.8 \times 10^{-8}$ |
| BOC – Phe-His-Sta-Leu-(2-amidomethylpyridine); | $2.5 \times 10^{-9}$ |
| BOC – Phe-His-Sta-Leu-(4-amido-1-benzylpiperidine); | $6 \times 10^{-11}$ |
| BOC – Phe-His-Sta-Leu-[N-(3-amidopropyl)diethanolamine]; | $5.2 \times 10^{-8}$ |

**Claims for the Contracting States: BE, CH, DE, FR, GB, IT, LI, LU, NL, SE**

1. A peptide of the formula:

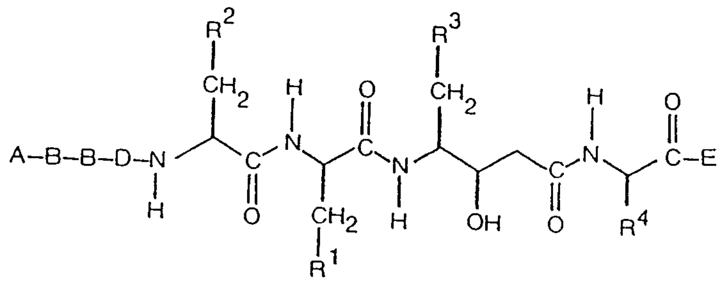

(I.)

wherein:

A is hydrogen;

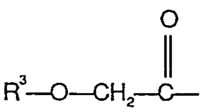 ; 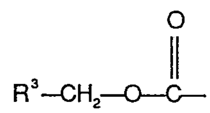 ;

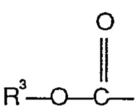 ; or 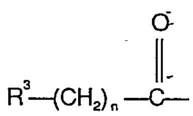

where n is 0 to 5 and $R^3$ has the same meaning as set out further below, and may additionally be hydrogen;

B is absent; glycyl; sarcosyl; or

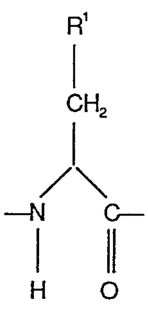

D is absent; or

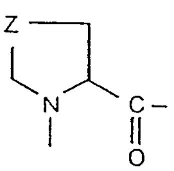

where Z is $(CH_2)_n$ and n is 1 or 2; or -S-;

$R^1$ is hydrogen; $C_{1-4}$ alkyl; hydroxy $C_{1-4}$ alkyl; phenyl; phenyl mono-substituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or $C_{3-7}$ cycloalkyl or phenyl mono-substituted with a member selected from the group consisting of methyl, trifluoromethyl, hydroxy, methoxy, fluoro, chloro, bromo, and iodo;

$R^4$ is hydrogen; or

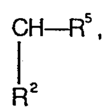

where $R^5$ is hydrogen; $C_{1-4}$ alkyl; hydroxy; or $C_{3-7}$ cycloalkyl; and

E is

(1) $-Y-(CH_2)_n-R^6$,

where

Y is -NH- or -O-;

n is 0 to 5; and

$R^6$ is hydrogen; hydroxy; $C_{1-4}$ alkyl; $C_{3-7}$ cycloalkyl; aryl; aryl substituted with up to five members independently selected from the group consisting of $C_{1-6}$ alkyl, trifluoromethyl, hydroxy, $C_{1-4}$ alkoxy, amino, mono- or di-$C_{1-4}$ alkylamino, and halo; am-

ino; mono-, di-, or tri-$C_{1-4}$ alkylamino; guanidyl; heterocyclic; or heterocyclic substituted with up to five members independently selected from the group consisting of $C_{1-6}$ alkyl; hydroxy, trifluoromethyl, $C_{1-4}$ alkoxy, halo, aryl, aryl $C_{1-4}$ alkyl, amino, and mono- or di-$C_{1-4}$ alkylamino;

(2) 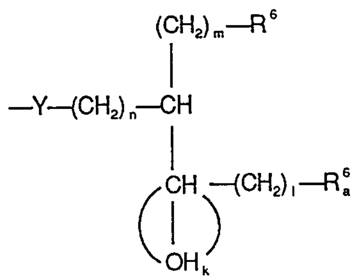

where
Y is as defined above;
n is 0 or 1;
k is 0 or 1;
l is 1 to 4;
m is 1 to 4; and
$R^6$ and $R^6_a$ may be the same or different and have the same meaning as $R^6$ above and $R^6_a$ may additionally be

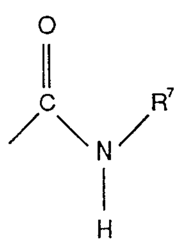 or 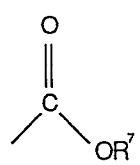

where $R^7$ is hydrogen or $C_{1-3}$ alkyl; or

(3) 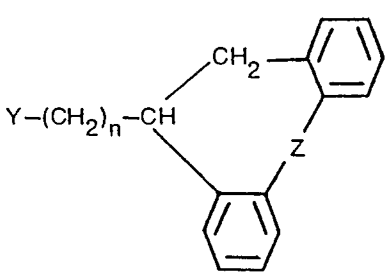

where
Y is as defined above; n is 0 or 1; and
Z is

(a) 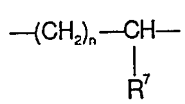

where
n is 0 or 1; and
$R^7$ is as defined above; or

(b) 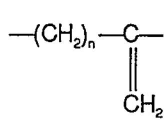

where
n is 0 or 1;
wherein all of the asymmetric carbon atoms have an S configuration, except for those in the B, D, and E substituents, which may have an S or R configuration; and a pharmaceutically acceptable salt thereof with the exception of peptides of formula (I) and their salts in which
$R^3$ is 2-propyl,
$R^4$ is methyl,
E is

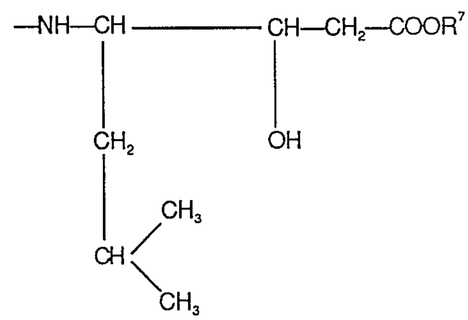

and B and D are absent.

2. A peptide according to Claim 1 wherein the peptide is a member selected from the group consisting of:

IBU - His-Pro-Phe-His-Sta-Leu-benzylamine;
IBU - His-Pro-Phe-His-Sta-Leu-2-phenylethylamide;
IBU - His-Pro-Phe-His-Sta-Leu-3-pehnylpropylamide;
IBU - His-Pro-Phe-His-Sta-Leu-1,2-diphenylethylamide;
BOC - Phe-His-Sta-Leu-(+)-1,2-diphenylethylamide;
BOC - Phe-His-Sta-Leu-(−)-1,2-diphenylethylamide;
BOC - Phe-His-Sta-Leu-benzylamide;
BOC - Phe-His-Sta-Leu-(+)-α-phenylethylamide;
BOC - Phe-His-Sta-Leu-(−)-α-phenylethylamide;
BOC - Phe-His-Sta-Leu-(+)-α-naphthylethylamide;
BOC - Phe-His-Sta-Leu-(−)-α-naphthylethylamide;
BOC - Phe-His-Sta-Leu-p-chlorobenzylamide;
BOC - Phe-His-Sta-Leu-p-methoxybenzylamide;
BOC -Phe-His-Sta-Leu-10,11-dihydro-5H-dibenzo[a,d]-cyclohepteneamide;
BOC - Phe-His-Sta-Leu-D,L-threo-1,2-diphenyl-2-hydroxyethylamide;
BOC - Phe-His-Sta-Leu-Sta;
BOC - Phe-His-AHPPA-Leu-benzylamide;
Acetyl - Phe-His-AHPPA-Leu-benzylamide;
BOC - Phe-His-Sta-Leu-(2-amidomethylpyridine);
BOC - Phe-His-Sta-Leu-(4-amidomethylpyridine);

BOC – Phe-His-Sta-Leu-(4-amido-1-benzylpiperidine);
BOC – Phe-His-Sta-Leu-[N-(3-amidopropyl)diethanolamine];
BOC – Phe-His-AHPPA-Leu-(2-amidomethylpyridine);
BOC – Phe-His-ACHPA[5]-Ile-(2-amidomethylpyridine);
IVA[6]-His-D-Pro-Phe-His-ACHPA-Ile-(2-amidomethylpyridine).

3. A pharmaceutical composition for treating renin-associated hypertension, comprising a pharmaceutical carrier and a therapeutically effective amount of a peptide of the formula:

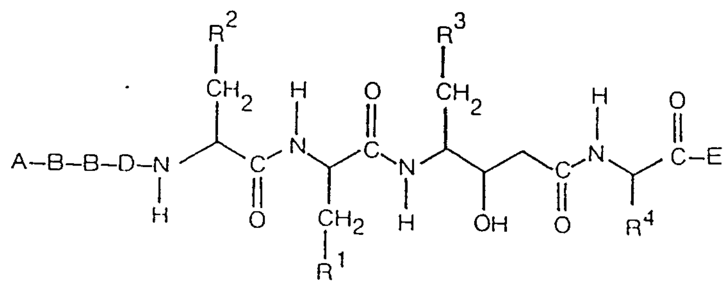 (I.)

wherein:

A is hydrogen;

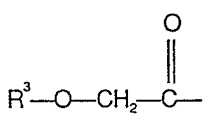 ; 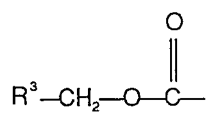 ;

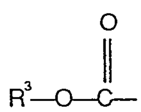 ; or 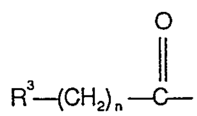

where n is 0 to 5 and $R^3$ has the same meaning as set out further below, and may additionally be hydrogen;

B is absent; glycyl; sarcosyl; or

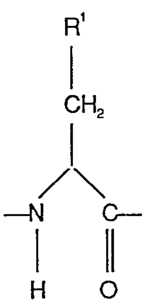

D is absent; or

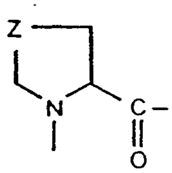

where Z is $(CH_2)_n$ and n is 1 or 2; or -S-;

$R^1$ is hydrogen; $C_{1-4}$ alkyl; hydroxy $C_{1-4}$ alkyl; phenyl; phenyl mono-subsituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or $C_{3-7}$ cycloalkyl or phenyl mono-substituted with a member selected from the group consisting of methyl, trifluoromethyl, hydroxy, methoxy, fluoro, chloro, bromo, and iodo;

$R^4$ is hydrogen; or

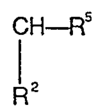 ,

where $R^5$ is hydrogen; $C_{1-4}$ alkyl; hydroxy; or $C_{3-7}$ cycloalkyl; and

E is

(1) $-Y-(CH_2)_n-R^6$,

where

Y is -NH- or -O-;

n is 0 to 5; and

$R^6$ is hydrogen; hydroxy; $C_{1-4}$alkyl; $C_{3-7}$cycloalkyl; aryl; aryl substituted with up to five members independently selected from the group consisting of $C_{1-6}$alkyl, trifluoromethyl, hydroxy, $C_{1-4}$ alkoxy, amino, mono- or di $C_{1-4}$alkylamino, and halo; amino; mono-, di-, or tri-$C_{1-4}$alkylamino; guanidyl; heterocyclic; or heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino;

(2) 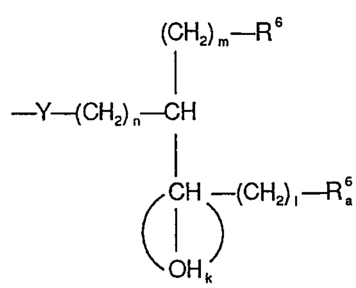

where
Y is as defined above;
n is 0 or 1;
k is 0 or 1;
l is 1 to 4;
m is 1 to 4; and
$R^6$ and $R^6_a$ may be the same or different and have the same meaning as $R^6$ above and $R^6_a$ may additionally be

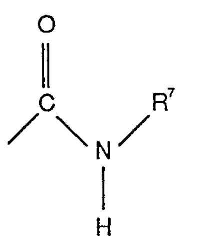

or

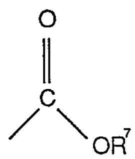

where $R^7$ is hydrogen or $C_{1-3}$alkyl; or

(3) 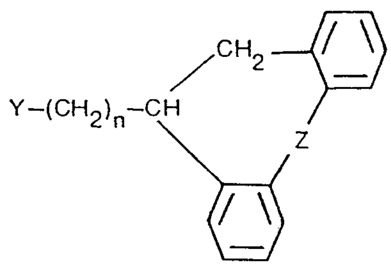

where
Y is as defined above; n is 0 or 1; and
Z is

(a) 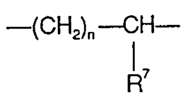

where
n is 0 or 1; and
$R^7$ is as defined above; or

(b) 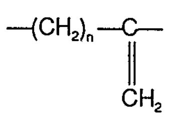

where
n is 0 or 1;
wherein all of the asymmetric carbon atoms have an S configuration, except for those in the B, D, and E substituents, which may have an S or R configuration; or of a pharmaceutically acceptable salt thereof with the exception of peptides of formula (I) and their salts in which
$R^3$ is 2-propyl,
$R^4$ is methyl,
E is

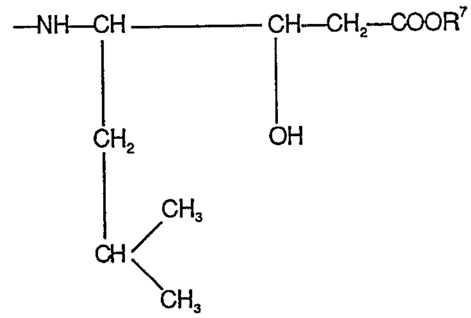

and B and D are absent.

4. A composition according to Claim 3 wherein the peptide is a member selected from the group consisting of:

IBU - His-Pro-Phe-His-Sta-Leu-benzylamine;
IBU - His-Pro-Phe-His-Sta-Leu-2-phenylethylamide;
IBU - His-Pro-Phe-His-Sta-Leu-3-phenylpropylamide;
IBU - His-Pro-Phe-His-Sta-Leu-1,2-diphenylethylamide;
BOC - Phe-His-Sta-Leu-(+)-1,2-diphenylethylamide;
BOC - Phe-His-Sta-Leu-(−)-1,2-diphenylethylamide;
BOC - Phe-His-Sta-Leu-benzylamide;
BOC - Phe-His-Sta-Leu-(+)-α-phenylethylamide;
BOC - Phe-His-Sta-Leu-(−)-α-phenylethylamide;
BOC - Phe-His-Sta-Leu-(+)-α-naphthylethylamide;
BOC - Phe-His-Sta-Leu-(−)-α-naphthylethylamide;
BOC - Phe-His-Sta-Leu-p-chlorobenzylamide;
BOC - Phe-His-Sta-Leu-p-methoxybenzylamide;
BOC - Phe-His-Sta-Leu-10,11-dihydro-5H-dibenzo[a,d]-cycloheptenеamide;
BOC - Phe-His-Sta-Leu-D,L-threo-1,2-diphenyl-2-hydroxyethylamide;
BOC - Phe-His-Sta-Leu-Sta;
BOC -Phe-His-AHPPA-Leu-benzylamide;
Acetyl- Phe-His-AHPPA-Leu-benzylamide;
BOC - Phe-His-Sta-Leu-(2-amidomethylpyridine);
BOC - Phe-His-Sta-Leu-(4-amidomethylpyridine);
BOC - Phe-His-Sta-Leu-(4-amido-1-benzylpiperidine);
BOC - Phe-His-Sta-Leu-[N-(3-amidopropyl)diethanolamine];
BOC - Phe-His-AHPPA-Leu-(2-amidomethylpyridine);
BOC - Phe-His-ACHPA-Ile-(2-amidomethylpyridine);

$IVA^6$-His-D-Pro-Phe-His-ACHPA-Ile-(2-amidomethylpyridine).

5. A pharmaceutical composition for treating renin-associated hyperaldosteronism, comprising a pharmaceutical carrfier and a therapeutically effective amount of a peptide of the formula

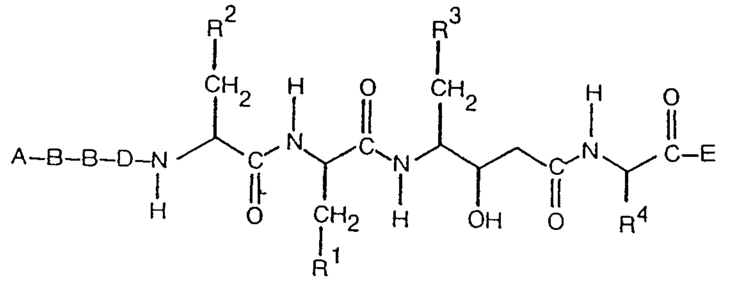

(I.)

wherein:

A is hydrogen;

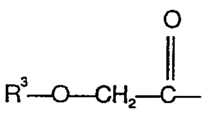 ; 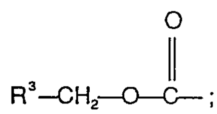 ;

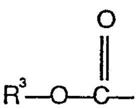 ; or 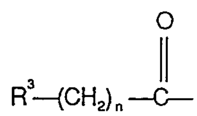

where n is 0 to 5 and $R^3$ has the same meaning as set out further below, and may additionally be hydrogen;

B is absent; glycyl; sarcosyl; or

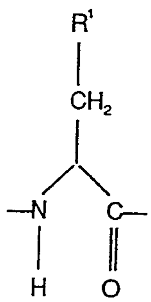

D is absent; or

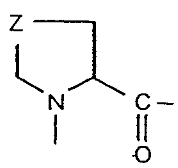

where Z is $(CH_2)_n$ and n is 1 or 2; or -S-;

$R^1$ is hydrogen; $C_{1-4}$ alkyl; hydroxy $C_{1-4}$ alkyl; phenyl; phenyl mono-subsituted with a member selected from the group consisting of methyl, trifloromethyl, hydroxy, methoxy, fluoro, chloro, bromo, and iodo; indolyl; 4-imidazolyl; amine $C_{1-4}$ alkyl; guanidyl $C_{2-3}$ alkyl; or methylthiomethyl;

$R^2$ is hydrogen $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or $C_{3-7}$ cycloalkyl or phenyl mono-substituted with a member selected from the group consisting of methyl, trifluoromethyl, hydroxy, methoxy, fluoro, chloro, bromo, and iodo;

$R^4$ is hydrogen; or

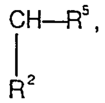

where $R^5$ is hydrogen; $C_{1-4}$ alkyl; hydroxy; or $C_{3-7}$ cycloalkyl; and

E is

(1) $-Y-(CH_2)_n-R^6$,

where

Y is -NH- or -O-;

n is 0 to 5; and

$R^6$ is hydrogen; hydroxy; $C_{1-4}$alkyl; $C_{3-7}$cycloalkyl; aryl; aryl substituted with up to five members independently selected from the group consisting of $C_{1-6}$alkyl, trifluoromethyl, hydroxy, $C_{1-4}$alkoxy, amino, mono- or di $C_{1-4}$alkylamino, and halo; amino; mono-, di-, or tri-$C_{1-4}$alkylamino; guanidyl; heterocyclic; or heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino;

(2) 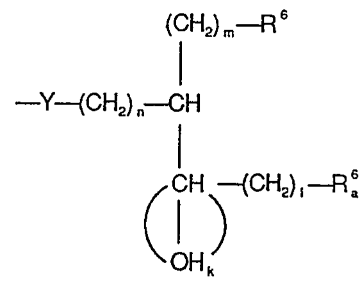

where

Y is as defined above;

n is 0 or 1;

k is 0 or 1;

l is 1 to 4;

m is 1 to 4; and

$R^6$ and $R^6_a$ may be the same or different and have the same meaning as $R^6$ above and $R^6_a$ may additionally be

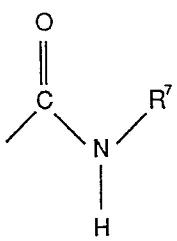
or
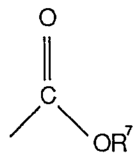

where $R^7$ is hydrogen or $C_{1-3}$alkyl; or

(3) 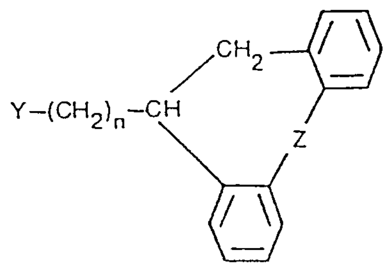

where
Y is as defined above; n is 0 or 1; and
Z is

(a) 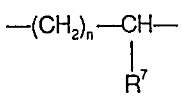

where
n is 0 or 1; and

$R^7$ is as defined above; or

(b) 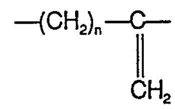

where
n is 0 or 1;
wherein all of the asymmetric carbon atoms have an S configuration, except for those in the B, D, and E substituents, which may have an S or R configuration; or of a pharmaceutically acceptable salt thereof with the exception of peptides of formula (I) and their salts in which
$R^3$ is 2-propyl,
$R^4$ is methyl,
E is

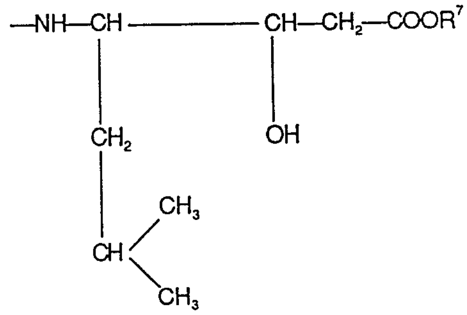

and B and D are absent.

**Claims for the Contracting State: AT**

1. A method of preparing a peptide of the formula:

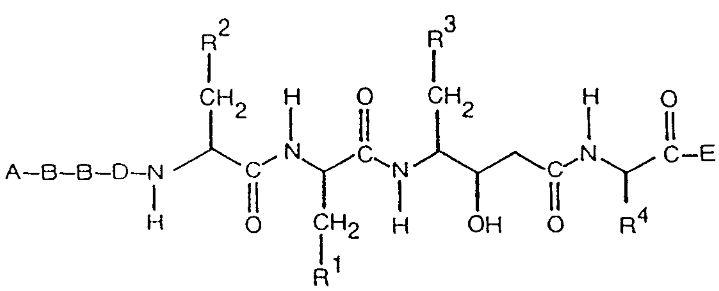
(I.)

wherein:
A is hydrogen;

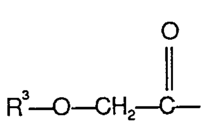; 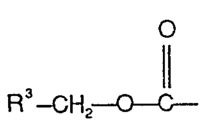;

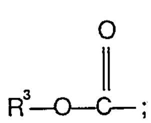; or 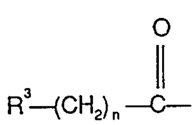

where n is 0 to 5 and $R^3$ has the same meaning as set out further below, and may additionally be hydrogen;

B is absent; glycyl; sarcosyl; or

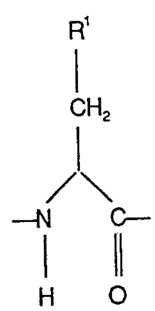

D is absent; or

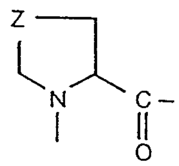

where Z is $(CH_2)_n$ and n is 1 or 2; or -S-;

$R^1$ is hydrogen; $C_{1-4}$ alkyl; hydroxy $C_{1-4}$ alkyl; phenyl; phenyl mono-substituted with a member selected from the group consisting of methyl, trifluoromethyl, hydroxy, methoxy, fluoro, chloro, bromo, and iodo; indolyl; 4-imidazolyl; amine $C_{2-4}$ alkyl; guanidyl $C_{2-3}$ alkyl; or methylthiomethyl;

$R^2$ is hydrogen $C_{1-4}$ alkyl; phenyl; phenyl mono-substituted with a member selected from the group consisting of methyl, trifluoromethyl, hydroxy, methoxy, fluoro, chloro, bromo, and iodo; or indolyl;

$R^3$ is $C_{3-6}$ alkyl; $C_{3-7}$ cycloalkyl; phenyl; or $C_{3-7}$ cycloalkyl or phenyl mono-substituted with a member selected from the group consisting of methyl, trifluoromethyl, hydroxy, methoxy, fluoro, chloro, bromo, and iodo;

$R^4$ is hydrogen; or

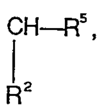

where $R^5$ is hydrogen; $C_{1-4}$ alkyl; hydroxy; or $C_{3-7}$ cycloalkyl; and

E is

(1) $-Y-(CH_2)_n-R^6$,

where

Y is -NH- or -O-;

n is 0 to 5; and

$R^6$ is hydrogen; hydroxy; $C_{1-4}$alkyl; $C_{3-7}$cycloalkyl; aryl; aryl substituted with up to five members independently selected from the group consisting of $C_{1-6}$alkyl, trifluoromethyl, hydroxy, $C_{1-4}$alkoxy, amino, mono- or di- $C_{1-4}$alkylamino, and halo; amino; mono-, di-, or tri-$C_{1-4}$alkylamino; guanidyl; heterocyclic; or heterocyclic substituted with up to five members independently selected from the group consisting of $C_{1-6}$ alkyl, hydroxy, trifluoromethyl, $C_{1-4}$ alkoxy, halo, aryl, aryl $C_{1-4}$ alkyl, amino, and mono- or di $C_{1-4}$ alkylamino;

(2) 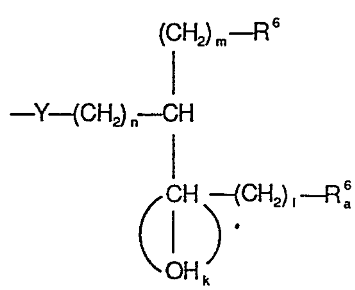

where

Y is as defined above;

n is 0 or 1;

k is 0 or 1;

l is 1 to 4;

m is 1 to 4; and

$R^6$ and $R^6_a$ may be the same or different and have the same meaning as $R^6$ above and $R^6_a$ may additionally be

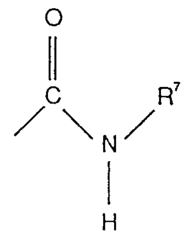 or 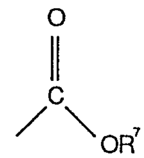

where $R^7$ is hydrogen or $C_{1-3}$alkyl; or

(3) 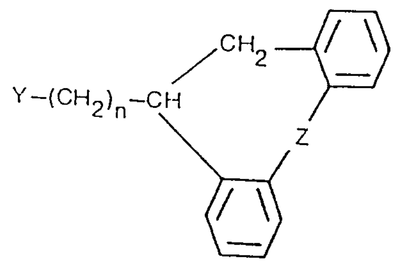

where

Y is as defined above; n is 0 or 1; and

Z is

(a) 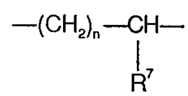

where

n is 0 or 1; and

$R^7$ is as defined above; or

(b) 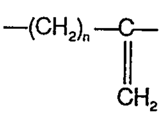

where

n is 0 or 1;

wherein all of the asymmetric carbon atoms have an S configuration, except for those in the B, D, and E substituents, which may have an S or R configuration; and a pharmaceutically acceptable salt thereof, said peptide being comprised of from five to seven amino acids identified as I through VII, amino acid (AA) I being at the C-terminus of said peptide, to which substituent E is attached, and amino acid (AA) VII being at the N-terminus of said peptide, to which substituents A–B–B–D are attached with the exception of peptides of formula (I) and their salts in which

$R^3$ is 2-propyl,

$R^4$ is methyl,

E is

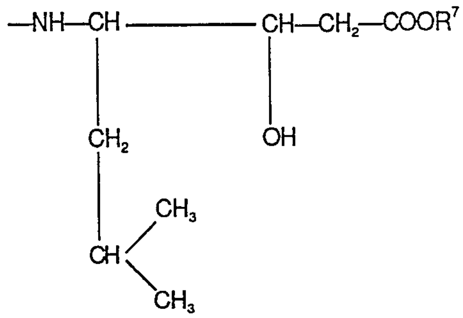

and B and D are absent, comprising the steps of:

(A) treating the desired ester or amide of the C-terminus amino acid (AA I) with the next adjacent amino acid (AA II) of said peptide, the amino group of said amino acid being protected by a protecting group, in the presence of a condensing agent, whereby a dipeptide of the two amino acids (AA I and II) is formed;

(B) deprotecting the dipeptide formed in Step (6A) by removing the protecting group from the amino group of AA II;

(C) treating the dipeptide of AA I and AA II with AA III, the amino group of which is protected by a protecting group, in the presence of a condensing agent, whereby a tripeptide of AA I, AA II and AA III is formed;

(D) deprotecting the tripeptide formed in Step (C) by removing the protecting group from the amino group of AA III;

(E) forming a quadripeptide up to a heptapeptide of AA I, through AA IV, AA V, AA VI or AA VII, by repeating the procedure of Step (C) using protected AA IV through protected AA VII;

(F) deprotecting the quadripeptide through heptapeptide formed in Step (E) to give the peptide of formula I wherein A is hydrogen; and optionally

(G) treating the quadripeptide through heptapeptide formed in Step (F) with

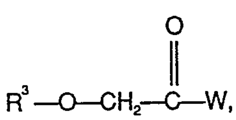, 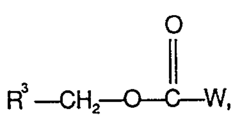,

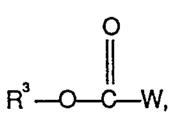, or 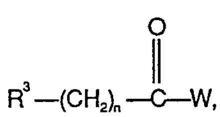,

where $R^3$ and n are as defined above and W is an acid halide, anhydride, or other carboxyl activating group, to give the peptide of formula I wherein A is other than hydrogen and optionally;

(H) treating the quadripeptide through heptapeptide formed in Steps (F) or (G) where an ester of AA I is employed with hydrazine to give the corresponding hydrazide, followed by treatment of said hydrazide with acidic nitrite to give the corresponding acyl azide, followed by treatment of said acyl azide with the appropriate amine compound to give the desired E substituent in the peptide of Formula I;

said method also comprising, where necessary, protection of sidechain substituents of the component amino acids AA I through AA VII, with deprotection being carried out as a final step; said method also comprising any combination of the steps set out above, whereby the amino acids I through VII and substituents A, B, D, and E are assembled in any desired order to prepare the peptide of formula I; and said method also comprising employment of the steps set out above in a solid phase sequential synthesis, whereby in the initial step the carboxyl group of the selected amino acid is bound to a synthetic resin substrate while the amino group of said amino acid is protected, followed by removal of the protecting group, the succeeding steps being as set out above, the peptide as it is assembled being attached to said synthetic resin substrate; followed by a step of removing the peptide of formula I from said synthetic resin substrate: (a) by strong acid cleavage to give E = OH; (b) by transesterification with a $C_{1-4}$ alkanol to give E = $O-C_{1-4}$alkyl (followed by hydrolysis to give E = OH); or (c) by ammonolysis with $NH_2R'$ where R' is hydrogen or $C_{1-4}$alkyl; and after removal of the peptide of Formula I from said synthetic resin substrate by transesterification to form the ester thereof as recited above, optionally the step of teating said ester thereof in accordance with the procedures described in Step (H) above to give the desired E substituent in the peptide of Formula I; removal of sidechain protecting groups being accomplished either before or after removal of the peptide of Formula I from said synthetic resin substrate.

2. The method of claim 1 for preparing a peptide from the group consisting of:

IBU - His-Pro-Phe-His-Sta-Leu-benzylamide;

IBU - His-Pro-Phe-His-Sta-Leu-2-phenylethylamide;

IBU - His-Pro-Phe-His-Sta-Leu-3-pehnylpropylamide;

IBU - His-Pro-Phe-His-Sta-Leu-1,2-diphenylethylamide;

BOC - Phe-His-Sta-Leu-(+)-1,2-diphenylethylamide;

BOC - Phe-His-Sta-Leu-(−)-1,2-diphenylethylamide;

BOC - Phe-His-Sta-Leu-benzylamide;

BOC - Phe-His-Sta-Leu-(+)-α-phenylethylamide;

BOC - Phe-His-Sta-Leu-(−)-α-phenylethylamide;

BOC - Phe-His-Sta-Leu-(+)-α-naphthylethylamide;

BOC - Phe-His-Sta-Leu-(−)-α-naphthylethylamide;

BOC - Phe-His-Sta-Leu-p-chlorobenzylamide;

BOC - Phe-His-Sta-Leu-p-methoxybenzylamide;

BOC - Phe-His-Sta-Leu-10,11-dihydro-5H-dibenzo[a,d]-cycloheptenemaide;

BOC - Phe-His-Sta-Leu-D,L-threo-1,2-diphenyl-2-hydroxyethylamide;
BOC - Phe-His-Sta-Leu-Sta;
BOC - Phe-His-AHPPA-Leu-benzylamide;
Acetyl - Phe-His-AHPPA-Leu-benzylamide;
BOC - Phe-His-Sta-Leu-(2-amidomethylpyridine);
BOC - Phe-His-Sta-Leu-(4-amidomethylpyridine);
BOC - Phe-His-Sta-Leu-(4-amido-1-benzylpiperidine);
BOC - Phe-His-Sta-Leu-[N-(3-amidopropyl)diethanolamine];
BOC - Phe-His-AHPPA-Leu-(2-amidomethylpyridine);
BOC - Phe-His-ACHPA-Ile-(2-amidomethylpyridine) or
IVA-His-D-Pro-Phe-His-ACHPA-Ile-(2-amidomethylpyridine).

## Patentansprüche

1. Ein Peptid der Formel

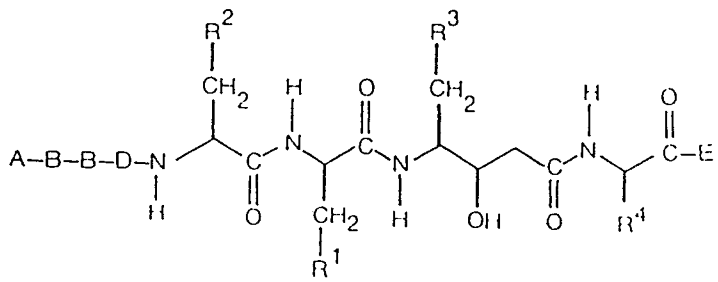

(I.)

worin
A Wasserstoff;

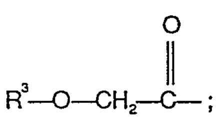 ; 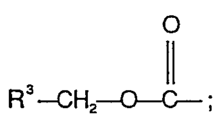 ;

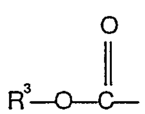 ; oder 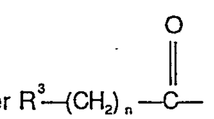

ist, worin n 0 bis 5 ist und $R^3$ die gleiche Bedeutung wie weiter unten angegeben hat, und zusätzlich Wasserstoff sein kann;
B fehlt; oder Glycyl; Sarcosyl; oder

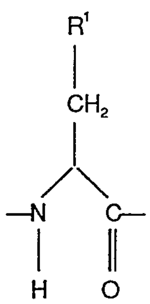

bedeutet;
D fehlt; oder

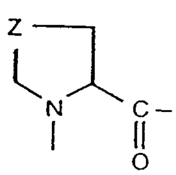

bedeutet, worin Z $(CH_2)_n$ ist, wobei n 1 oder 2 ist; oder -S- bedeutet;
$R^1$ Wasserstoff; $C_{1-4}$-Alkyl; Hydroxy-$C_{1-4}$-alkyl; Phenyl; oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; Indolyl; 4-Imidazolyl; Amin-$C_{2-4}$-alkyl; Guanidyl-$C_{2-3}$-alkyl; oder Methylthiomethyl; bedeutet;
$R^2$ Wasserstoff, $C_{1-4}$Alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; oder Indolyl bedeutet,
$R^3$ $C_{3-6}$-Alkyl; $C_{3-7}$-Cycloalkyl; Phenyl; oder $C_{3-7}$-Cycloalkyl oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist, bedeutet;
$R^4$ Wasserstoff; oder

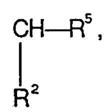

worin $R^5$ Wasserstoff ist; $C_{1-4}$-Alkyl; Hydroxy; oder $C_{3-7}$-Cycloalkyl bedeutet, und
E (1) $-Y-(CH_2)_n-R^6$,
worin
Y -NH- oder -O- ist;
n 0 bis 5 ist; und
$R^6$ Wasserstoff; Hydroxy; $C_{1-4}$-Alkyl; $C_{3-7}$-Cycloalkyl; Aryl; Aryl, das durch bis zu fünf Glieder substituiert ist, welche unabhängig voneinander aus der Gruppe ausgewählt sind, die aus $C_{1-6}$-Alkyl, Trifluormethyl, Hydroxy, $C_{1-4}$-Alkoxy, Amino, Mono- oder Di-$C_{1-4}$-alkylamino und Halogen besteht; Amino; Mono-, Di- oder Tri-$C_{1-4}$-alkylamino; Guanidyl; Heterozyklen; oder Heterozyklen, die durch bis zu fünf Glieder substituiert sind, welche unabhängig voneinander aus der Gruppe ausgewählt sind, die aus $C_{1-6}$-Alkyl, Hydroxy, Trifluormethyl, $C_{1-4}$-Alkoxy, Halogen, Aryl, Aryl-$C_{1-4}$-

alkyl, Amino und Mono- oder Di-$C_{1-4}$-alkylamino besteht; ist,

(2) 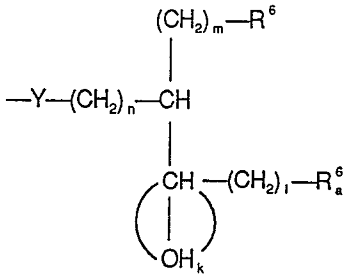

worin
Y wie oben definiert ist;
n 0 oder 1 ist;
k 0 oder 1 ist;
l 1 bis 4 ist;
m 1 bis 4 ist; und
$R^6$ und $R^6_a$ gleich verschieden sein können und die gleiche Bedeutung wie $R^6$ oben haben und $R^6_a$ zusätzlich

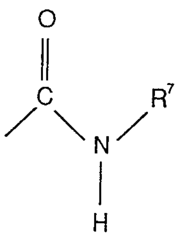 oder 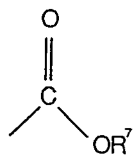

sein kann,
worin $R^7$ Wasserstoff oder $C_{1-3}$-Alkyl ist; oder

(3) 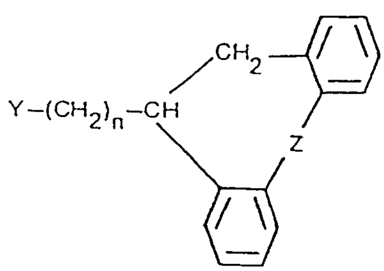

worin
Y wie oben definiert ist;
n 0 oder 1 ist; und
Z

(a) 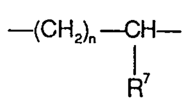

ist, worin
n 0 oder 1 ist; und
$R^7$ wie oben definiert ist; oder

(b) 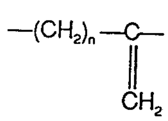

ist, worin
n 0 oder 1 ist; bedeutet, wobei alle asymmetrischen Kohlenstoffatome eine S-Konfiguration, ausgenommen solche in den B-, D- und E-Substituenten, die eine S- oder R-Konfiguration haben können, aufweisen; und ein pharmazeutisch annehmbares Salz davon, mit der Ausnahme von Peptiden der Formel (I) und ihren Salzen, worin
$R^3$ 2-Propyl ist,
$R^4$ Methyl ist,

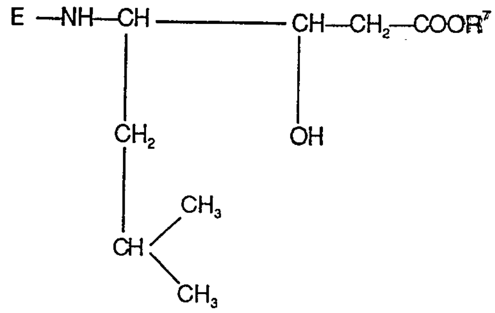

ist, und B und D fehlen.

2. Ein Peptid nach Anspruch 1, worin das Peptid ein Glied ist, das aus der Gruppe ausgewählt ist, die aus:
IBU - His-Pro-Phe-His-Sta-Leu-Benzylamid;
IBU - His-Pro-Phe-His-Sta-Leu-2-Phenylethylamid;
IBU - His-Pro-Phe-His-Sta-Leu-3-Phenylpropylamid;
IBU - His-Pro-Phe-His-Sta-Leu-1,2-Diphenylethylamid;
BOC - Phe-His-Sta-Leu-(+)-1,2-Diphenylethylamid;
BOC - Phe-His-Sta-Leu-(−)-1,2-Diphenylethylamid;
BOC - Phe-His-Sta-Leu-Benzylamid;
BOC - Phe-His-Sta-Leu-(+)-α-Phenylethylamid;
BOC - Phe-His-Sta-Leu-(−)-α-Phenylethylamid;
BOC - Phe-His-Sta-Leu-(+)-α-Naphthylethylamid;
BOC - Phe-His-Sta-Leu-(−)-α-Naphthylethylamid;
BOC - Phe-His-Sta-Leu-p-Chlorbenzylamid;
BOC - Phe-His-Sta-Leu-p-Methoxybenzylamid;
BOC - Phe-His-Sta-Leu-10,11-Dihydro-5H-dibenzo-[a,d]-cycloheptenamid;
BOC - Phe-His-Sta-Leu-D,L-Threo-1,2-diphenyl-2-hydroxyethylamid;
BOC - Phe-His-Sta-Leu-Sta;
BOC - Phe-His-AHPPA-Leu-Benzylamid;
Acetyl - Phe-His-AHPPA-Leu-Benzylamid;
BOC - Phe-His-Sta-Leu-(2-Amidomethylpyridin);
BOC - Phe-His-Sta-Leu-(4-Amidomethylpyridin);
BOC - Phe-His-Sta-Leu-(4-Amido-1-benzylpiperidin);
BOC - Phe-His-Sta-Leu-[N-(3-Amidopropyl)diethanolamin];
BOC - Phe-His-AHPPA-Leu-(2-Amidomethylpyridin);
BOC - Phe-His-ACHPA-Ile-(2-Amidomethylpyridin)
IVA-His-D-Pro-Phe-His-ACHPA-Ile-(2-Amidomethylpyridin) besteht.

3. Eine pharmazeutische Zusammensetzung für die Behandlung von reninassoziierter Hypertension, enthaltend einen pharmazeutischen Träger und eine therapeutisch wirksame Menge eines Peptids der Formel:

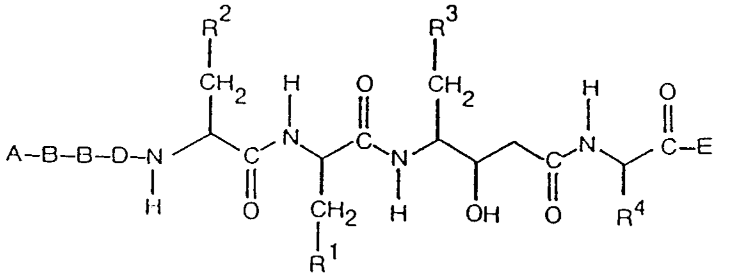
(I.)

worin
A Wasserstoff;

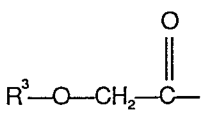 ; 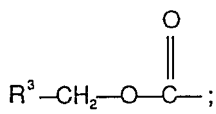 ;

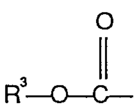 ; oder 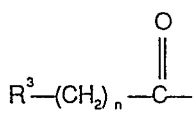

ist, worin n 0 bis 5 ist und $R^3$ die gleiche Bedeutung wie weiter unten angegeben hat, und zusätzlich Wasserstoff sein kann;
B fehlt; oder Glycyl; Sarcosyl; oder

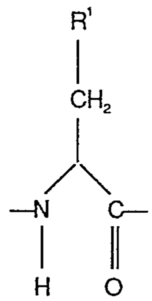

bedeutet;
D fehlt; oder

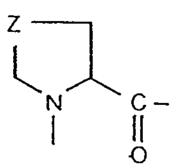

bedeutet, worin Z $(CH_2)_n$ ist, wobei n 1 oder 2 ist; oder -S- bedeutet;
$R^1$ Wasserstoff; $C_{1-4}$-Alkyl; Hydroxy-$C_{1-4}$-alkyl; Phenyl; oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; Indolyl; 4-Imidazolyl; Amin-$C_{2-4}$-alkyl; Guanidyl-$C_{2-3}$-alkyl; oder Methylthiomethyl; bedeutet;
$R^2$ Wasserstoff, $C_{1-4}$ Alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; oder Indolyl bedeutet,
$R^3$ $C_{3-6}$-Alkyl; $C_{3-7}$-Cycloalkyl; Phenyl; oder $C_{3-7}$-Cycloalkyl oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist, bedeutet;
$R^4$ Wasserstoff; oder

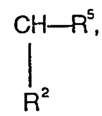

worin $R^5$ Wasserstoff ist; $C_{1-4}$-Alkyl; Hydroxy; oder $C_{3-7}$-Cycloalkyl bedeutet, und
E (1) $-Y-(CH_2)_n-R^6$,
worin
Y -NH- oder -O- ist;
n 0 bis 5 ist; und
$R^6$ Wasserstoff; Hydroxy; $C_{1-4}$-Alkyl; $C_{3-7}$-Cycloalkyl; Aryl; Aryl, das durch bis zu fünf Glieder substituiert ist, welche unabhängig voneinander aus der Gruppe ausgewählt sind, die aus $C_{1-6}$-Alkyl, Trifluormethyl, Hydroxy, $C_{1-4}$-Alkoxy, Amino, Mono- oder Di-$C_{1-4}$-alkylamino und Halogen besteht; Amino; Mono-, Di- oder Tri-$C_{1-4}$-alkylamino; Guanidyl; Heterozyklen; oder Heterozyklen, die durch bis zu fünf Glieder substituiert sind, welche unabhängig voneinander aus der Gruppe ausgewählt sind, die aus $C_{1-6}$-Alkyl, Hydroxy, Trifluormethyl, $C_{1-4}$-Alkoxy, Halogen, Aryl, Aryl-$C_{1-4}$-alkyl, Amino und Mono- oder Di-$C_{1-4}$-alkylamino besteht; ist,

(2) 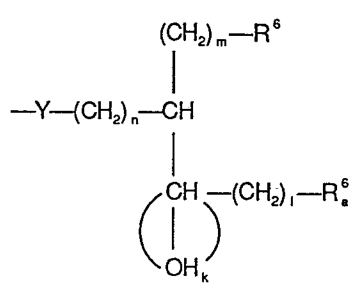

worin
Y wie oben definiert ist;
n 0 oder 1 ist;
k 0 oder 1 ist;
l 1 bis 4 ist;

m 1 bis 4 ist; und
$R^6$ und $R^6_a$ gleich oder verschieden sein können und die gleiche Bedeutung wie $R^6$ oben haben und $R^6_a$ zusätzlich

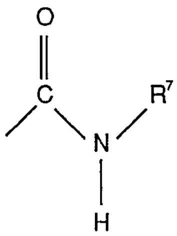

oder

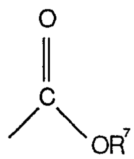

sein kann,
worin $R^7$ Wasserstoff oder $C_{1-3}$-Alkyl ist; oder

(3) 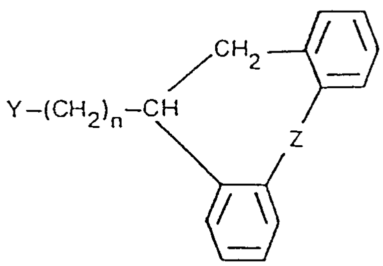

worin
Y wie oben definiert ist;
n 0 oder 1 ist; und
Z

(a) 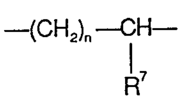

ist, worin
n 0 oder 1 ist; und
$R^7$ wie oben definiert ist; oder

(b) 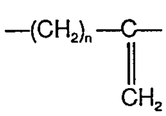

ist, worin
n 0 oder 1 ist; bedeutet, wobei alle asymmetrischen Kohlenstoffatome eine S-Konfiguration, ausgenommen solche in den B-, D- und E-Substituenten, die eine S- oder R-Konfiguration haben können, aufweisen; oder eines pharmazeutisch annehmbaren Salzes davon, mit der Ausnahme von Peptiden der Formel (I) und ihren Salzen, worin
$R^3$ 2-Propyl ist,
$R^4$ Methyl ist,

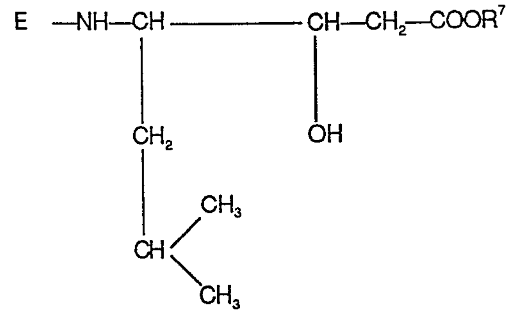

ist, und B und D fehlen.

4. Eine Zusammensetzung nach Anspruch 3, worin das Peptid ein Glied ist, das aus der Gruppe ausgewählt ist, die aus:
IBU - His-Pro-Phe-His-Sta-Leu-Benzylamid;
IBU - His-Pro-Phe-His-Sta-Leu-2-Phenylethylamid;
IBU - His-Pro-Phe-His-Sta-Leu-3-Phenylpropylamid;
IBU - His-Pro-Phe-His-Sta-Leu-1,2-Diphenylethylamid;
BOC - Phe-His-Sta-Leu-(+)-1,2-Diphenylethylamid;
BOC - Phe-His-Sta-Leu-(−)-1,2-Diphenylethylamid;
BOC - Phe-His-Sta-Leu-Benzylamid;
BOC - Phe-His-Sta-Leu-(+)-α-Phenylethylamid;
BOC - Phe-His-Sta-Leu-(−)-α-Phenylethylamid;
BOC - Phe-His-Sta-Leu-(+)-α-Naphthylethylamid;
BOC - Phe-His-Sta-Leu-(−)-α-Naphthylethylamid;
BOC - Phe-His-Sta-Leu-p-Chlorbenzylamid;
BOC - Phe-His-Sta-Leu-p-Methoxybenzylamid;
BOC - Phe-His-Sta-Leu-10,11-Dihydro-5H-dibenzo-[a,d]-cycloheptenamid;
BOC - Phe-His-Sta-Leu-D,L-Threo-1,2-diphenyl-2-hydroxyethylamid;
BOC - Phe-His-Sta-Leu-Sta;
BOC - Phe-His-AHPPA-Leu-Benzylamid;
Acetyl - Phe-His-AHPPA-Leu-Benzylamid;
BOC - Phe-His-Sta-Leu-(2-Amidomethylpyridin);
BOC - Phe-His-Sta-Leu-(4-Amidomethylpyridin);
BOC - Phe-His-Sta-Leu-(4-Amido-1-benzylpiperidin);
BOC - Phe-His-Sta-Leu-[N-(3-Amidopropyl)diethanolamin];
BOC - Phe-His-AHPPA-Leu-(2-Amidomethylpyridin);
BOC - Phe-His-ACHPA-Ile-(2-Amidomethylpyridin)
IVA-His-D-Pro-Phe-His-ACHPA-Ile-(2-Amidomethylpyridin) besteht.

5. Eine pharmazeutische Zusammensetzung für die Behandlung von reninassoziierter Hyperaldosteronismus, enthaltend einen pharmazeutischen Träger und eine therapeutisch wirksame Menge eines Peptids der Formel:

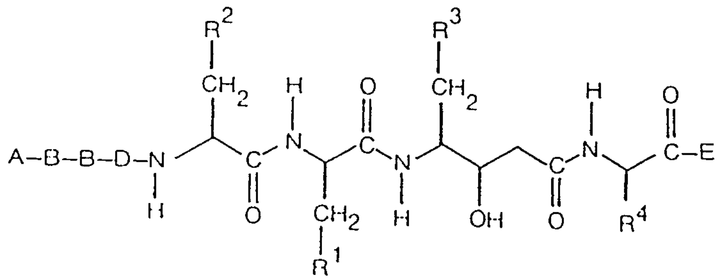

(I.)

worin

A Wasserstoff;

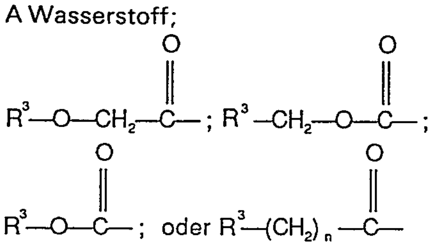

ist, worin n 0 bis 5 ist und $R^3$ die gleiche Bedeutung wie weiter unten angegeben hat, und zusätzlich Wasserstoff sein kann;

B fehlt; oder Glycyl; Sarcosyl; oder

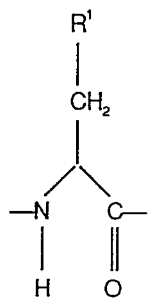

bedeutet; D fehlt; oder

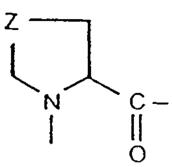

bedeutet, worin Z $(CH_2)_n$ ist, wobei n 1 oder 2 ist; oder -S- bedeutet;

$R^1$ Wasserstoff; $C_{1-4}$-Alkyl; Hydroxy-$C_{1-4}$-alkyl; Phenyl; oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; Indolyl; 4-Imidazolyl; Amin-$C_{2-4}$-alkyl; Guanidyl-$C_{2-3}$-alkyl; oder Methylthiomethyl; bedeutet;

$R^2$ Wasserstoff, $C_R{}^2$ Wasserstoff; $C_{1-4}$-Alkyl; Phenyl; Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist; oder Indolyl bedeutet,

$R^3$ $C_{3-6}$-Alkyl; $C_{3-7}$-Cycloalkyl; Phenyl; oder $C_{3-7}$-Cycloalkyl oder Phenyl, das durch ein Glied monosubstituiert ist, welches aus der aus Methyl, Trifluormethyl, Hydroxy, Methoxy, Fluor, Chlor, Brom und Iod bestehenden Gruppe ausgewählt ist, bedeutet;

$R^4$ Wasserstoff; oder

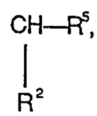

worin $R^5$ Wasserstoff ist; $C_{1-4}$-Alkyl; Hydroxy; oder $C_{3-7}$-Cycloalkyl bedeutet, und

E (1) $-Y-(CH_2)_n-R^6$,

worin

Y -NH- oder -O- ist;

n 0 bis 5 ist; und

$R^6$ Wasserstoff; Hydroxy; $C_{1-4}$-Alkyl; $C_{3-7}$-Cycloalkyl; Aryl; Aryl, das durch bis zu fünf Glieder substituiert ist, welche unabhängig voneinander aus der Gruppe ausgewählt sind, die aus $C_{1-6}$-Alkyl, Trifluormethyl, Hydroxy, $C_{1-4}$-Alkoxy, Amino, Mono- oder Di-$C_{1-4}$-alkylamino und Halogen besteht; Amino; Mono-, Di- oder Tri-$C_{1-4}$-alkylamino; Guanidyl; Heterozyklen; oder Heterozyklen, die durch bis zu fünf Glieder substituiert sind, welche unabhängig voneinander aus der Gruppe ausgewählt sind, die aus $C_{1-6}$-Alkyl, Hydroxy, Trifluormethyl, $C_{1-4}$-Alkoxy, Halogen, Aryl, Aryl-$C_{1-4}$-alkyl, Amino und Mono- oder Di-$C_{1-4}$-alkylamino besteht; ist,

(2) 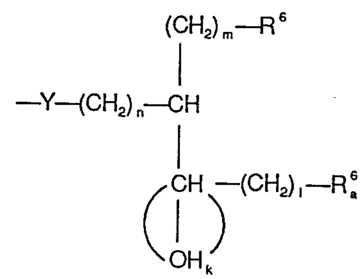

worin

Y wie oben definiert ist;

n 0 oder 1 ist;

k 0 oder 1 ist;

l 1 bis 4 ist;

m 1 bis 4 ist; und

$R^6$ und $R^6_a$ gleich oder verschieden sein können und die gleiche Bedeutung wie $R^6$ oben haben und $R^6_a$ zusätzlich

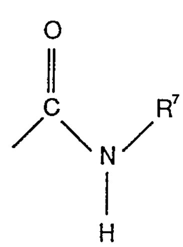 oder 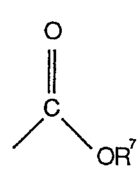

sein kann,
worin $R^7$ Wasserstoff oder $C_{1-3}$-Alkyl ist; oder

(3) 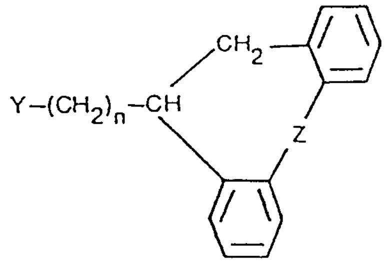

worin
Y wie oben definiert ist;
n 0 oder 1 ist; und
Z

(a) 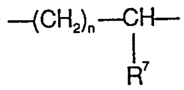

ist, worin
n 0 oder 1 ist; und
$R^7$ wie oben definiert ist; oder

(b) 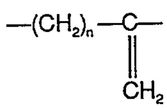

ist, worin
n 0 oder 1 ist; bedeutet, wobei alle asymmetrischen Kohlenstoffatome eine S-Konfiguration, ausgenommen solche in den B-, D- und E-Substituenten, die eine S- oder R-Konfiguration haben können, aufweisen; oder eines pharmazeutisch annehmbaren Salzes davon,
mit der Ausnahme von Peptiden der Formel (I) und ihren Salzen, worin
$R^3$ 2-Propyl ist,
$R^4$ Methyl ist,
E

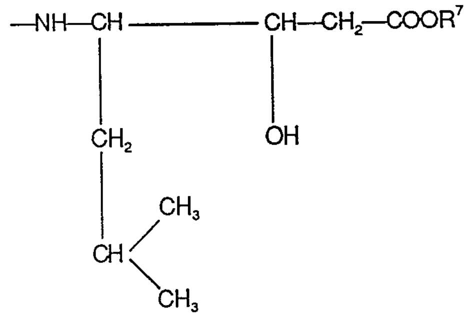

ist, und
B und D fehlen.

**Patentansprüche AT**

1. Ein Verfahren zur Herstellung eines Peptids der Formel:

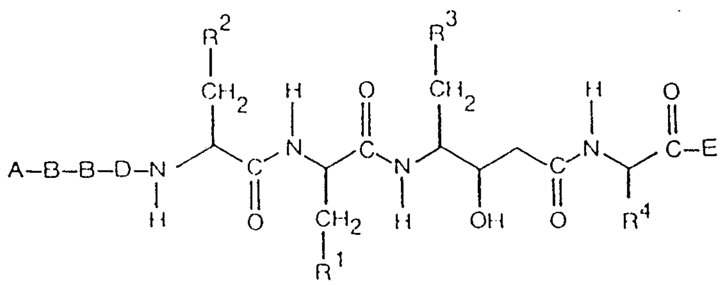
(I.)

worin
A Wasserstoff;

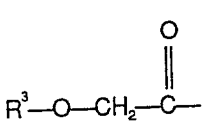 ; 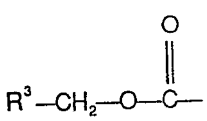 ;

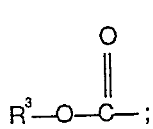 ; oder 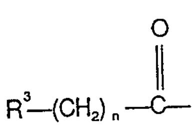

ist, worin n 0 bis 5 ist und $R^3$ die gleiche Bedeutung wie weiter unten angegeben hat, und zusätzlich Wasserstoff sein kann;

B fehlt; oder Glycyl; Sarcosyl; oder

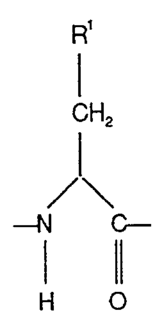

bedeutet;
D fehlt; oder

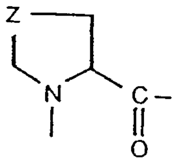

bedeutet, worin Z $(CH_2)_n$ ist, wobei n 1 oder 2 ist; oder -S- bedeutet;
$R^1$ Wasserstoff; $C_{1-4}$-Alkyl; Hydroxy-$C_{1-4}$-alkyl; Phenyl; oder Phenyl, das durch ein Glied monosubstituiert ist, welches aus der aus Methyl, Trifluormethyl, Hydroxy, Methoxy, Fluor, Chlor, Brom und lod bestehenden Gruppe ausgewählt ist; Indolyl; 4-Imidazolyl; Amin-$C_{2-4}$-alkyl; Guanidyl-$C_{2-3}$-alkyl; oder Methylthiomethyl; bedeutet;
$R^2$ Wasserstoff; $C_{1-4}$-Alkyl; Phenyl; Phenyl, das durch ein Glied monosubstituiert ist, welches aus der aus Methyl, Trifluormethyl, Hydroxy, Methoxy, Fluor, Chlor, Brom und lod bestehenden Gruppe ausgewählt ist; oder Indolyl bedeutet,
$R^3$ $C_{3-6}$-Alkyl; $C_{3-7}$-Cycloalkyl; Phenyl; oder $C_{3-7}$-Cycloalkyl oder Phenyl, das durch ein Glied monosubstituiert ist, welches aus der aus Methyl, Trifluormethyl, Hydroxy, Methoxy, Fluor, Chlor, Brom und lod bestehenden Gruppe ausgewählt ist, bedeutet;
$R^4$ Wasserstoff; oder

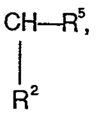

worin $R^5$ Wasserstoff ist;
$C_{1-4}$-Alkyl; Hydroxy; oder $C_{3-7}$-Cycloalkyl bedeutet, und
E (1) $-Y-(CH_2)_n-R^6$,
worin
Y -NH- oder -O- ist;
n 0 bis 5 ist; und
$R^6$ Wasserstoff; Hydroxy; $C_{1-4}$-Alkyl; $C_{3-7}$-Cycloalkyl; Aryl; Aryl, das durch bis zu fünf Glieder substituiert ist, welche unabhängig voneinander aus der Gruppe ausgewählt sind, die aus $C_{1-6}$-Alkyl, Trifluormethyl, Hydroxy, $C_{1-4}$-Alkoxy, Amino, Mono- oder Di-$C_{1-4}$-alkylamino und Halogen besteht; Amino; Mono-, Di- oder Tri-$C_{1-4}$-alkylamino; Guanidyl; Heterozyklen; oder Heterozyklen, die durch bis zu fünf Glieder substituiert sind, welche unabhängig voneinander aus der Gruppe ausgewählt sind, die aus $C_{1-6}$-Alkyl, Hydroxy, Trifluormethyl, $C_{1-4}$-Alkoxy, Halogen, Aryl, Aryl-$C_{1-4}$-alkyl, Amino und Mono- oder Di-$C_{1-4}$-alkylamino besteht; ist,

(2) 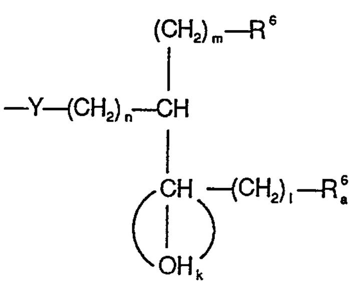

worin
Y wie oben definiert ist;
n 0 oder 1 ist;
k 0 oder 1 ist;
l 1 bis 4 ist;
m 1 bis 4 ist; und
$R^6$ und $R^6_a$ gleich oder verschieden sein können und die gleiche Bedeutung wie $R^6$ oben haben und $R^6_a$ zusätzlich

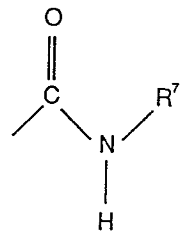 oder 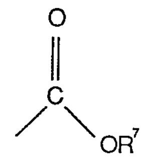

sein kann,
worin $R^7$ Wasserstoff oder $C_{1-3}$-Alkyl ist; oder

(3) 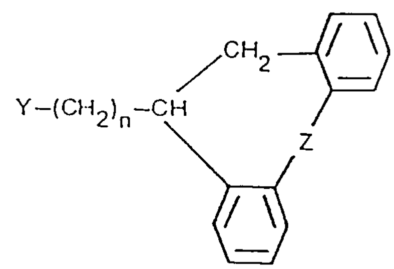

worin
Y wie oben definiert ist;
n 0 oder 1 ist; und
Z

(a) 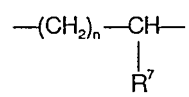

ist, worin
n 0 oder 1 ist; und
$R^7$ wie oben definiert ist; oder

(b) 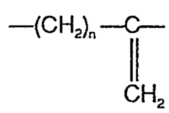

ist, worin
n 0 oder 1 ist; bedeutet, wobei alle asymmetrischen Kohlenstoffatome eine S-Konfiguration, ausgenommen solche in den B-, D- und E-Substituenten, die eine S- oder R-Konfiguration haben können, aufweisen; und eines pharmazeutisch annehmbaren Salzes davon, wobei das genannte Peptid aus fünf bis sieben Aminosäuren besteht, die mit I bis VII bezeichnet sind, Aminosäure (AA) I sich am C-Terminus des genannten Peptids befindet, an welchen der Substituent E gebunden ist, und Aminosäure (AA) VII sich am N-Terminus des genannten Peptids befindet, an den die Substituenten A-B-B-D gebunden sind, mit der Ausnahme von Peptiden der Formel (I) und ihren Salzen, worin
$R^3$ 2-Propyl ist,

$R^4$ Methyl ist,
E

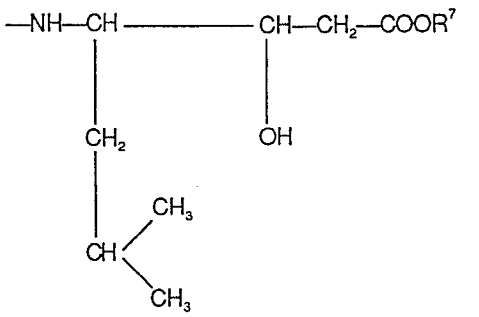

ist, und
B und D fehlen, umfassend die Stufen:
(A) der Behandlung des gewünschten Esters oder Amids der C-Terminus-Aminosäure (AA I) mit der nächsten benachbarten Aminosäure (AA II) des genannten Peptids, wobei die Aminogruppe der genannten Aminosäure durch eine Schutzgruppe geschützt ist, in Gegenwart eines Kondensationsmittels, wobei ein Dipeptid der zwei Aminosäuren (AA I und II) gebildet wird;
(B) der Freisetzung des in Stufe (A) gebildeten Dipeptids durch Entfernung der Schutzgruppe von der Aminogruppe von AA II;
(C) der Behandlung des Dipeptids aus AA I und AA II mit AA III, deren Aminogruppe durch eine Schutzgruppe geschützt ist,
in Gegenwart eines Kondensationsmittels, wobei ein Tripeptid von AA I, AA II und AA III gebildet wird;
(D) der Freisetzung des in Stufe (C) gebildeten Tripeptids durch Entfernung der Schutzgruppe von der Aminogruppe von AA III;
(E) der Bildung eines Quadripeptids bis zu einem Heptapeptid von AA I bis AA IV, AA V, AA VI oder AA VII, durch Wiederholung der Verfahrensweise von Stufe (C) unter Verwendung geschützter AA IV bis geschützter AA VII;
(F) der Freisetzung des in Stufe (E) gebildeten Quadripeptids bis Heptapeptids unter Bildung des Peptids der Formel I, worin A Wasserstoff ist; und gegebenenfalls
(G) der Behandlung des in Stufe (F) gebildeten Quadripeptids bis Heptapeptids mit

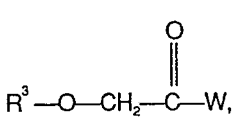, 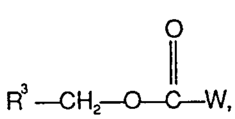,

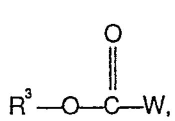, oder 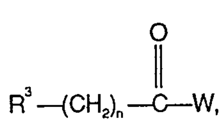,

worin $R^3$ und n wie oben definiert sind, und W eine Säurehalogenid-, -anhydrid- oder andere carboxylaktivierende Gruppe ist, unter Bildung des Peptids der Formel I, worin A eine von Wasserstoff verschiedene Bedeutung hat, und gegebenenfalls
(H) der Behandlung des in den Stufen (F) oder (G) gebildeten Quadripeptids bis Heptapeptids, wobei ein Ester von AA I zusammen mit Hydrazin unter Bildung des entsprechenden Hydrazids angewendet wird, mit anschliessender Behandlung des genannten Hydrazids mit saurem Nitrit unter Bildung des entsprechenden Acylazids, gefolgt von der Behandlung des genannten Acylazids mit der geeigneten Aminverbindung, um den gewünschten E-Substituenten in dem Peptid der Formel I zu erhalten;
wobei das genannte Verfahren erforderlichenfalls auch den Schutz von Seitenkettensubstituenten der Komponenten Aminosäuren AA I bis AA VII, mit Ausführung der Freisetzung als eine Endstufe, umfasst; wobei das genannte Verfahren auch irgendeine Kombination der oben angeführten Stufen umfasst, wobei die Aminosäuren I bis VII und Substituenten A, B, D und E in irgendeiner gewünschten Reihenfolge aneinandergefügt werden, um das Peptid der Formel I herzustellen; und wobei das genannte Verfahren auch die Anwendung der oben angegebenen Stufen in einer Festphasenfolgesynthese umfasst, wobei in der Anfangsstufe die Carboxylgruppe der gewählten Aminosäure an ein Substrat aus synthetischem Harz gebunden wird, während die Aminogruppe der genannten Aminosäure geschützt ist, gefolgt von der Entfernung der Schutzgruppe, und wobei die folgenden Stufen wie oben angegeben ausgeführt werden, wobei das Peptid, während es zusammengefügt wird, an das genannte Substrat aus synthetischem Harz gebunden vorliegt; worauf eine Stufe der Entfernung des Peptids der Formel I aus dem genannten Substrat aus synthetischem Harz; (a) durch Spaltung mit starker Säure unter Bildung von E=OH; (b) durch Umesterung mit einem $C_{1-4}$-Alkanol unter Bildung von E = O–$C_{1-4}$-Alkyl (gefolgt von einer Hydrolyse unter Bildung von E = OH); oder (c) durch Ammonolyse mit $NH_2R'$, worin R′ Wasserstoff oder $C_{1-4}$-Alkyl ist, vorgenommen wird; und wobei nach Entfernung des Peptids der Formel I aus dem genannten Substrat aus synthetischem Harz durch Umesterung unter Bildung des Esters davon, wie oben angegeben, gegebenenfalls die Stufe der Behandlung des genannten Esters davon nach den in Stufe (H) oben beschriebenen Verfahrensweisen, unter Bildung des gewünschten Substituenten E in dem Peptid der Formel I, angewendet wird; wobei die Entfernung von Seitenkettenschutzgruppen entweder vor oder nach Entfernung des Peptids der Formel I aus dem genannten Substrat aus synthetischem Harz erfolgt.

2. Das Verfahren des Anspruchs 1 zur Herstellung eines Peptids aus der Gruppe, die aus:
IBU -
His-Pro-Phe-His-Sta-Leu-Benzylamid;
IBU -
His-Pro-Phe-His-Sta-Leu-2-Phenylethylamid;
IBU-
His-Pro-Phe-His-Sta-Leu-3-Phenylpropylamid;

IBU – His-Pro-Phe-His-Sta-Leu-1,2-Diphenylethylamid;
BOC – Phe-His-Sta-Leu-(+)-1,2-Diphenylethylamid;
BOC – Phe-His-Sta-Leu-(−)-1,2-Diphenylethylamid;
BOC – Phe-His-Sta-Leu-Benzylamid;
BOC – Phe-His-Sta-Leu-(+)-α-Phenylethylamid;
BOC – Phe-His-Sta-Leu-(−)-α-Phenylethylamid;
BOC – Phe-His-Sta-Leu-(+)-α-Naphthylethylamid;
BOC – Phe-His-Sta-Leu-(−)-α-Naphthylethylamid;
BOC – Phe-His-Sta-Leu-p-Chlorbenzylamid;
BOC – Phe-His-Sta-Leu-p-Methoxybenzylamid;
BOC – Phe-His-Sta-Leu-10,11-Dihydro-5H-dibenzo-[a,d]-cycloheptenamid;
BOC – Phe-His-Sta-Leu-D,L-Threo-1,2-diphenyl-2-hydroxyethylamid;
BOC – Phe-His-Sta-Leu-Sta;
BOC – Phe-His-AHPPA-Leu-Benzylamid;
Acetyl – Phe-His-AHPPA-Leu-Benzylamid;
BOC – Phe-His-Sta-Leu-(2-Amidomethylpyridin);
BOC – Phe-His-Sta-Leu-(4-Amidomethylpyridin);
BOC – Phe-His-Sta-Leu-(4-Amido-1-benzylpiperidin);
BOC – Phe-His-Sta-Leu-[N-(3-Amidopropyl)diethanolamin];
BOC – Phe-His-AHPPA-Leu-(2-Amidomethylpyridin);
BOC – Phe-His-ACHPA-Ile-(2-Amidomethylpyridin); und
IVA-His-D-Pro-Phe-His-ACHPA-Ile-(2-Amidomethylpyridin) besteht.

## Revendications

1. Peptide de formule

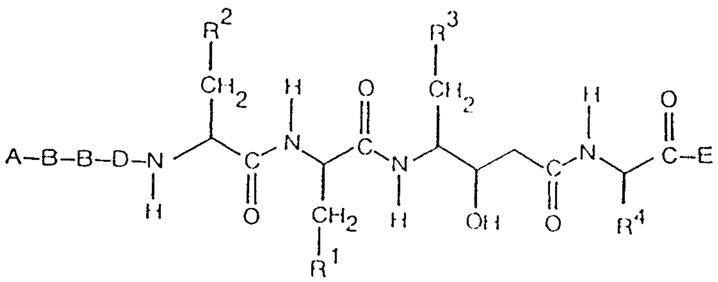

(I.)

où
A est un hydrogène:

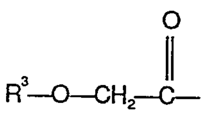 ; 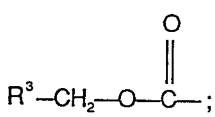 ;

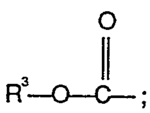 ; ou 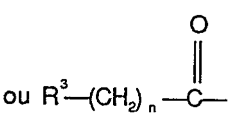

où n vaut de 0 à 5 et R3 a la même signification que celle donnée plus loin, et peut en outre être un hydrogène:
B est absent; un glycyle: un sarcosyle; ou

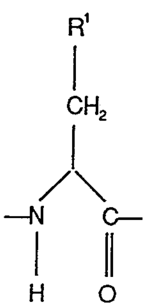

D est absent; ou

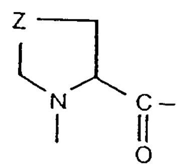

où Z est $(CH_2)_n$ et n vaut 1 ou 2; ou -S-;
R1 est un hydrogène; un alcoyle en C1 à C4; un hydroxy-alcoyle en C1 à C4; un phényle; un phényle mono-substitué par un membre choisi dans le groupe constitué par méthyle, trifluorométhyle, hydroxy, méthoxy, fluoro, chloro, bromo et iodo; un indolyle; un 4-imidazolyle; un aminealcoyle en C1 à C4; un guanidyl-alcoyle en C2 à C3; ou un méthylthiométhyle;
R2 est un hydrogène; un alcoyle en C1 à C4; un phényle; un phényle monosubstitué par un membre choisi dans le groupe constitué par méthyle, trifluorométhyle, hydroxy, méthoxy, fluoro, chloro, bromo et iodo; ou indolyle;
R3 est un alcoyle en C3 à C6; un cycloalcoyle en C3 à C7; un phényle; ou un cycloalcoyle en C3 à C7 ou un phényle mono-substitué par un membre choisi dans le groupe constitué par méthyle, trifluorométhyle, hydroxy, méthoxy, fluoro, chloro, bromo et iodo;
R4 est un hydrogène:

ou 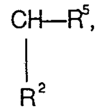

où R5 est un hydrogène; un alcoyle en C1 à C4; un hydroxy; ou un cycloalcoyle en C3 à C7; et
E est (1) -Y-$(CH_2)_n$-R6
où
Y est -NH- ou -O-;
n vaut de 0 à 5; et
R6 est un hydrogène; un hydroxy; un alcoyle en C1 à C4; un cycloalcoyle en C3 à C7; un aryle; un aryle substitué par jusqu'à 5 membres choisis indépendamment dans le groupe constitué par alcoyle en C1 à C6, trifluorométhyle, hydroxy, alcoxy en C1 à C4, amino, mono-ou di-alcoyle en C1 à C4-amino, et halo; amino; mono-, di- ou tri-alcoyle en C1 à C4-amino; guanidyle; un hétérocycle; ou un hétérocycle substitué par jusqu'à 5 membres choisis indépendamment dans le groupe constitué par alcoyle en C1 à C6, hydroxy, trifluorométhyle, alcoxy en C1 à C4, halo, aryle, aryl-alcoyle en C1 à C4, amino, et mono- ou di-alcoyle en C1 à C4-amino;

(2) 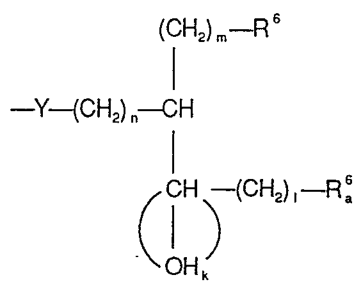

où
Y est tel que défini ci-dessus:
n vaut 0 ou 1;
k vaut 0 ou 1;
l vaut de 1 à 4;
m vaut de 1 à 4; et
$R^6$ et $R^6_a$ peuvent être semblables ou différents et ont la même signification que $R^6$ ci-dessus et $R^6_a$ peut en outre être

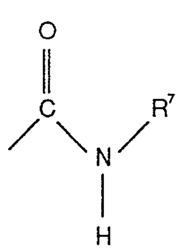 ou 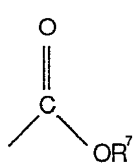

où R7 est un hydrogène ou un alcoyle en C1 à C3;

(3) 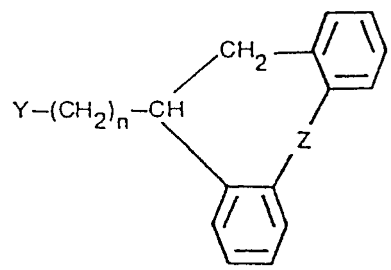

où
Y est tel que défini ci-dessus;
n vaut 0 ou 1; et
Z est

(a) 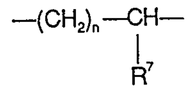

où
n vaut 0 ou 1; et
R7 est tel que défini ci-dessus: ou

(b) 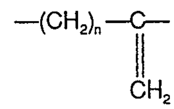

où
n vaut 0 ou 1;
où tous les atomes de carbone asymétriques ont une configuration S, sauf ceux qui sont dans les substituants B, D et E, qui peuvent avoir une configuration S ou R;
et un de ses sels pharmaceutiquement acceptables, à l'exception toutefois des peptides de formule (I) et de leurs sels, dans laquelle
$R^3$ est 2 propyle,
$R^4$ est méthyle,
E est

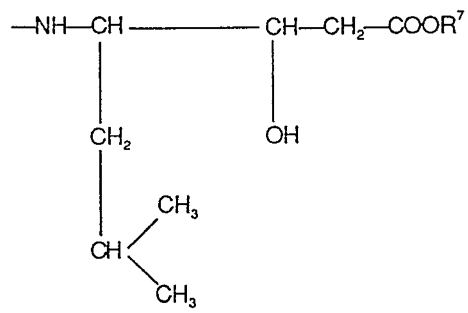

et B et D sont absents.

2. Peptide selon la revendication 1 où le peptide est un membre choisi dans le groupe constitué par
IBU -
His-Pro-Phe-His-Sta-Leu-benzylamide;
IBU -
His-Pro-Phe-His-Sta-Leu-2-phényléthylamide;
IBU -
His-Pro-Phe-His-Sta-Leu-3-phénylpropylamide;
IBU -
His-Pro-Phe-His-Sta-Leu-1,2-diphényléthylamide;

BOC - Phe-His-Sta-Leu-(+)-1,2-diphényléthylamide;
BOC - Phe-His-Sta-Leu-(−)-1,2-diphényléthylamide;
BOC - Phe-His-Sta-Leu-benzylamide;
BOC - Phe-His-Sta-Leu-(+)-α-phényléthylamide;
BOC - Phe-His-Sta-Leu-(−)-α-phényléthylamide;
BOC - Phe-His-Sta-Leu-(+)-α-naphthyléthylamide;
BOC - Phe-His-Sta-Leu-(−)-α-naphthyléthylamide;
BOC - Phe-His-Sta-Leu-p-chlorbenzylamide;
BOC - Phe-His-Sta-Leu-p-méthoxybenzylamide;
BOC - Phe-His-Sta-Leu-10,11-dihydro-5H-dibenzo-[a,d]-cycloheptèneamide;
BOC - Phe-His-Sta-Leu-D,L-thréo-1,2-diphényl-2-hydroxyéthylamide;
BOC - Phe-His-Sta-Leu-Sta;
BOC - Phe-His-AHPPA-Leu-benzylamide;
Acétyl - Phe-His-AHPPA-Leu-benzylamide;
BOC - Phe-His-Sta-Leu-(2-amidométhylpyridine);
BOC - Phe-His-Sta-Leu-(4-amidométhylpyridine);
BOC - Phe-His-Sta-Leu-(4-amido-1-benzylpipéridine);
BOC - Phe-His-Sta-Leu-[N-(3-amidopropyl)diéthanolamine];
BOC - Phe-His-AHPPA-Leu-(2-amidométhylpyridine);
BOC - Phe-His-ACHPA-Ile-(2-amidométhylpyridine);
IVA-His-D-Pro-Phe-His-ACHPA-Ile-(2-amidométhylpyridine).

3. Composition pharmaceutique pour traiter l'hypertension associée à la rennine, comprenant un support pharmaceutique et une quantité thérapeutiquement efficace d'un peptide de formule

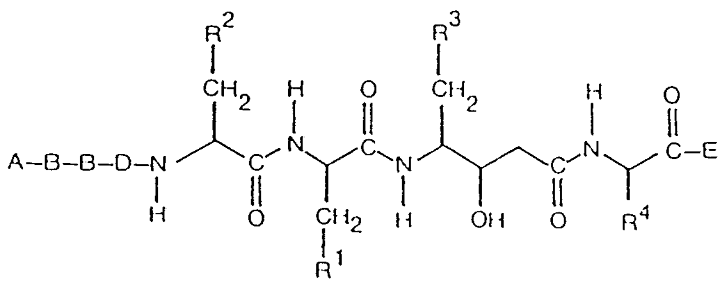

(I.)

où
A est un hydrogène;

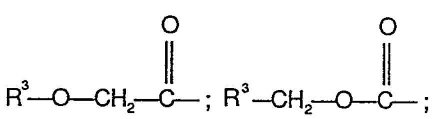

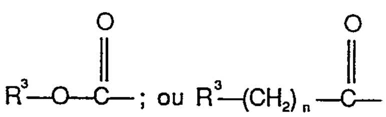

où n vaut de 0 à 5 et R3 a la même signification que celle donnée plus loin, et peut en outre être un hydrogène:
B est absent; un glycyle: un sarcosyle; ou

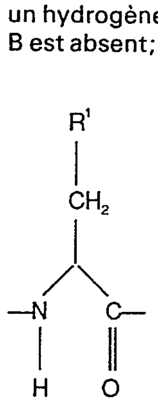

D est absent; ou

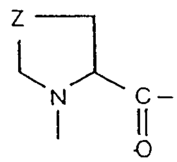

où Z est $(CH_2)_n$ et n vaut 1 ou 2; ou -S-;
R1 est un hydrogène; un alcoyle en C1 à C4; un hydroxy-alcoyle en C1 à C4; un phényle; un phényle mono-substitué par un membre choisi dans le groupe constitué par méthyle, trifluorométhyle, hydroxy, méthoxy, fluoro, chloro, bromo et iodo; un indolyle; un 4-imidazolyle; un aminealcoyle en C1 à C4; un guanidyl-alcoyle en C2 à C3; ou un méthylthiométhyle;
R2 est un hydrogène; un alcoyle en C1 à C4; un phényle; un phényle monosubstitué par un membre choisi dans le groupe constitué par méthyle, trifluorométhyle, hydroxy, méthoxy, fluoro, chloro, bromo et iodo; ou indolyle;
R3 est un alcoyle en C3 à C6; un cycloalcoyle en C3 à C7; un phényle; ou un cycloalcoyle en C3 à C7 ou un phényle mono-substitué par un membre choisi dans le groupe constitué par méthyle, trifluorométhyle, hydroxy, méthoxy, fluoro, chloro, bromo et iodo;
R4 est un hydrogène:

ou 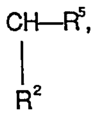

où R5 est un hydrogène; un alcoyle en C1 à C4; un hydroxy; ou un cycloalcoyle en C3 à C7; et

E est (1) $-Y-(CH_2)_n-R6$

où

Y est $-NH-$ ou $-O-$;

n vaut de 0 à 5; et

R6 est un hydrogène; un hydroxy; un alcoyle en C1 à C4; un cycloalcoyle en C3 à C7; un aryle; un aryle substitué par jusqu'à 5 membres choisis indépendamment dans le groupe constitué par alcoyle en C1 à C6, trifluorométhyle, hydroxy, alcoxy en C1 à C4, amino, mono- ou di-alcoyle en C1 à C4-amino, et halo; amino; mono-, di- ou tri-alcoyle en C1 à C4-amino; guanidyle; un hétérocycle; ou un hétérocycle substitué par jusqu'à 5 membres choisis indépendamment dans le groupe constitué par alcoyle en C1 à C6, hydroxy, trifluorométhyle, alcoxy en C1 à C4, halo, aryle, aryl-alcoyle en C1 à C4, amino, et mono- ou di-alcoyle en C1 à C4-amino;

(2) 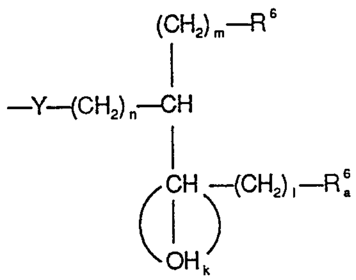

où

Y est tel que défini ci-dessus:

n vaut 0 ou 1;

k vaut 0 ou 1;

l vaut de 1 à 4;

m vaut de 1 à 4; et

$R^6$ et $R^6_a$ peuvent être semblables ou différents et ont la même signification que $R^6$ ci-dessus et $R^6_a$ peut en outre être

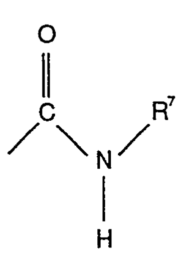 ou 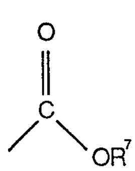

où R7 est un hydrogène ou un alcoyle en C1 à C3;

(3) 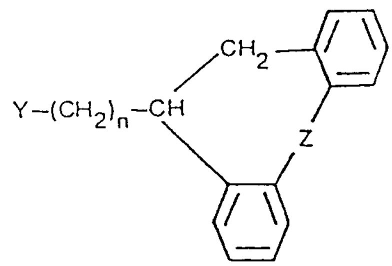

où

Y est tel que défini ci-dessus;

n vaut 0 ou 1; et

Z est

(a) 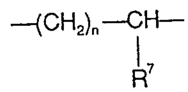

où

n vaut 0 ou 1; et

R7 est tel que défini ci-dessus: ou

(b) 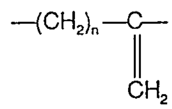

où

n vaut 0 ou 1;

où tous les atomes de carbone asymétriques ont une configuration S, sauf ceux qui sont dans les substituants B, D et E, qui peuvent avoir une configuration S ou R;

et d'un de ses sels pharmaceutiquement acceptables, à l'exception toutefois des peptides de formule (I) et de leurs sels, dans laquelle

$R^3$ est 2 propyle,

$R^4$ est méthyle,

E est

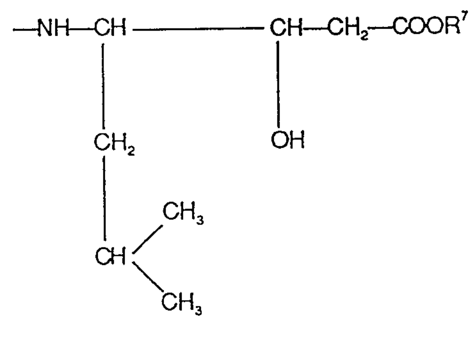

et B et D sont absents.

4. Composition selon la revendication 3 où le peptide est un membre choisi dans le groupe constitué par:

IBU - His-Pro-Phe-His-Sta-Leu-benzylamide;

IBU - His-Pro-Phe-His-Sta-Leu-2-phényléthylamide;

IBU - His-Pro-Phe-His-Sta-Leu-3-phénylpropylamide;

IBU - His-Pro-Phe-His-Sta-Leu-1,2-diphényléthylamide;

BOC- Phe-His-Sta-Leu-(+)-1,2-diphényléthylamide;
BOC - Phe-His-Sta-Leu-(−)-1,2-diphényléthylamide;
BOC - Phe-His-Sta-Leu-benzylamide;
BOC - Phe-His-Sta-Leu-(+)-α-phényléthylamide;
BOC - Phe-His-Sta-Leu-(−)-α-phényléthylamide;
BOC - Phe-His-Sta-Leu-(+)-α-naphthyléthylamide;
BOC - Phe-His-Sta-Leu-(−)-α-naphthyléthylamide;
BOC - Phe-His-Sta-Leu-p-chlorbenzylamide;
BOC - Phe-His-Sta-Leu-p-méthoxybenzylamide;
BOC - Phe-His-Sta-Leu-10,11-dihydro-5H-dibenzo-[a,d]-cycloheptèneamide;
BOC - Phe-His-Sta-Leu-D,L-threo-1,2-diphényl-2-hydroxyéthylamide;
BOC - Phe-His-Sta-Leu-Sta;
BOC - Phe-His-AHPPA-Leu-benzylamide;
Acétyl - Phe-His-AHPPA-Leu-benzylamide;
BOC - Phe-His-Sta-Leu-(2-amidométhylpyridine);
BOC - Phe-His-Sta-Leu-(4-amidométhylpyridine);
BOC - Phe-His-Sta-Leu-(4-amido-1-benzylpipéridine);
BOC - Phe-His-Sta-Leu-[N-(3-amidopropyl)diéthanolamine];
BOC - Phe-His-AHPPA-Leu-(2-amidométhylpyridine);
BOC - Phe-His-ACHPA-Ile-(2-amidométhylpyridine);
IVA-His-D-Pro-Phe-His-ACHPA-Ile-(2-amidométhylpyridine).

5. Composition pharmaceutique pour traiter l'hyperaldostéronisme associée à la rennine, comprenant un support pharmaceutique et une quantité thérapeutiquement efficace d'un peptide de formule

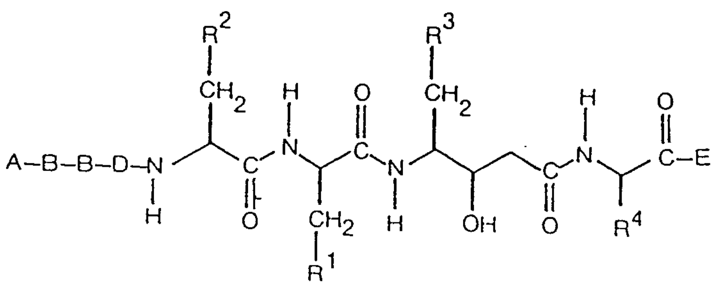

(I.)

où
A est un hydrogène;

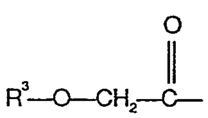
;
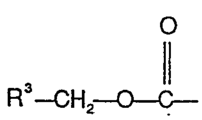
;

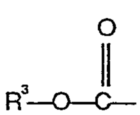
; ou
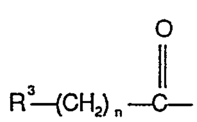

où n vaut de 0 à 5 et R3 a la même signification que celle donnée plus loin, et peut en outre être un hydrogène:
B est absent; un glycyle: un sarcosyle; ou

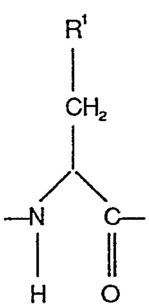

D est absent; ou

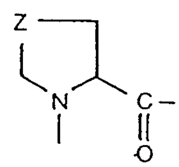

où Z est $(CH_2)_n$ et n vaut 1 ou 2; ou -S-;
R1 est un hydrogène; un alcoyle en C1 à C4; un hydroxy-alcoyle en C1 à C4; un phényle; un phényle mono-substitué par un membre choisi dans le groupe constitué par méthyle, trifluorométhyle, hydroxy, méthoxy, fluoro, chloro, bromo et iodo; un indolyle; un 4-imidazolyle; un aminealcoyle en C1 à C4; un guanidyl-alcoyle en C2 à C3; ou un méthylthiométhyle;
R2 est un hydrogène; un alcoyle en C1 à C4; un phényle; un phényle monosubstitué par un membre choisi dans le groupe constitué par méthyle, trifluorométhyle, hydroxy, méthoxy, fluoro, chloro, bromo et iodo; ou indolyle;
R3 est un alcoyle en C3 à C6; un cycloalcoyle en C3 à C7; un phényle; ou un cycloalcoyle en C3 à C7 ou un phényle mono-substitué par un membre choisi dans le groupe constitué par méthyle, trifluorométhyle, hydroxy, méthoxy, fluoro, chloro, bromo et iodo;
R4 est un hydrogène:

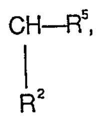

où R5 est un hydrogène; un alcoyle en C1 à C4; un hydroxy; ou un cycloalcoyle en C3 à C7; et
E est (1) -Y-$(CH_2)_n$-R6
où
Y est-NH-ou-O-;
n vaut de 0 à 5; et
R6 est un hydrogène; un hydroxy; un alcoyle en C1 à C4; un cycloalcoyle en C3 à C7; un aryle; un aryle substitué par jusqu'à 5 membres choisis indépendamment dans le groupe con constitué par alcoyle en C1 à C6, trifluorométhyle, hydroxy, alcoxy en C1 à C4, amino, mono- ou di-alcoyle en C1 à C4-amino, et halo; amino; mono-, di- ou tri-alcoyle en C1 à C4-amino; guanidyle; un hétérocycle; ou un hétérocycle substitué par jusqu'à 5 membres choisis indépendamment dans le groupe constitué par alcoyle en C1 à C6, hydroxy, trifluorométhyle, alcoxy en C1 à C4, halo, aryle, aryl-alcoyle en C1 à C4, amino, et mono- ou di-alcoyle en C1 à C4-amino;

(2) 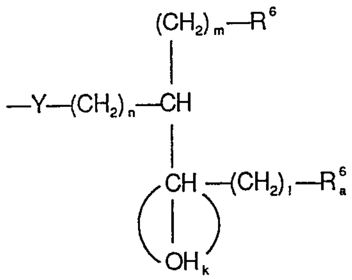

où
Y est tel que défini ci-dessus:
n vaut 0 ou 1;
k vaut 0 ou 1;
l vaut de 1 à 4;
m vaut de 1 à 4; et
$R^6$ et $R^6_a$ peuvent être semblables ou différents et ont la même signification que $R^6$ ci-dessus et $R^6_a$ peut en outre être

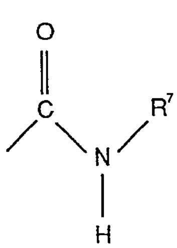 ou 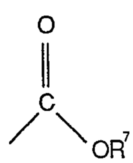

où R7 est un hydrogène ou un alcoyle en C1 à C3;

(3) 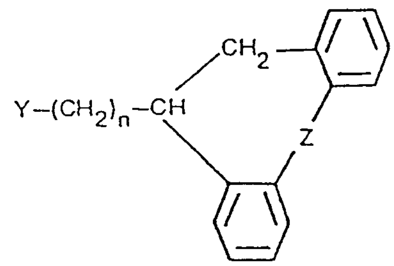

où
Y est tel que défini ci-dessus;
n vaut 0 ou 1; et
Z est

(a) 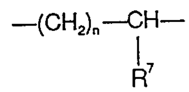

où
n vaut 0 ou 1; et
R7 est tel que défini ci-dessus: ou

(b) 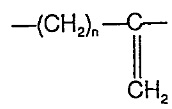

où
n vaut 0 ou 1;
où tous les atomes de carbone asymétriques ont une configuration S, sauf ceux qui sont dans les substituants B, D et E, qui peuvent avoir une configuration S ou R;
et d'un de ses sels pharmaceutiquement acceptables, à l'exception toutefois des peptides de formule (I) et de leurs sels, dans laquelle
$R^3$ est 2 propyle,
$R^4$ est méthyle,
E est

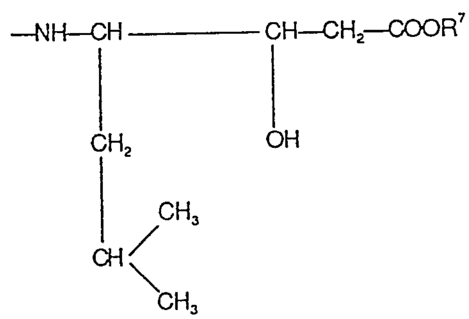

et B et D sont absents.

**Revendications AT**

1. Procédé de préparation d'un peptide de formule

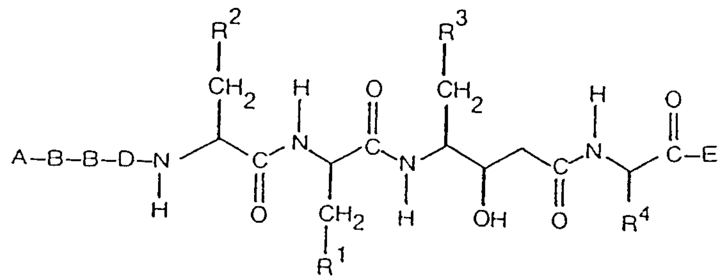

(I.)

où

A est un hydrogène;

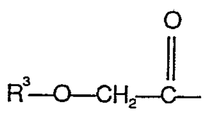 ; 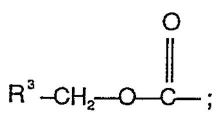 ;

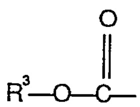 ; ou 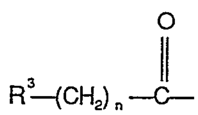

où n vaut de 0 à 5 et R3 a la même signification que celle donnée plus loin, et peut en outre être un hydrogène:

B est absent; un glycyle; un sarcosyle; ou

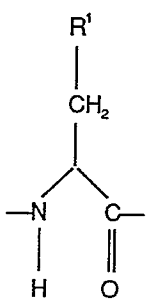

D est absent; ou

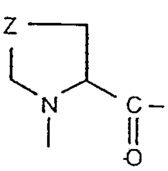

où Z est $(CH_2)_n$ et n vaut 1 ou 2; ou -S-;

R1 est un hydrogène; un alcoyle en C1 à C4; un hydroxy-alcoyle en C1 à C4; un phényle; un phényle mono-substitué par un membre choisi dans le groupe constitué par méthyle, trifluorométhyle, hydroxy, méthoxy, fluoro, chloro, bromo et iodo; un indolyle; un 4-imidazolyle; un aminealcoyle en C1 à C4; un guanidyl-alcoyle en C2 à C3; ou un méthylthiométhyle;

R2 est un hydrogène; un alcoyle en C1 à C4; un phényle; un phényle monosubstitué par un membre choisi dans le groupe constitué par méthyle, trifluorométhyle, hydroxy, méthoxy, fluoro, chloro, bromo et iodo; ou indolyle;

R3 est un alcoyle en C3 à C6; un cycloalcoyle en C3 à C7; un phényle; ou un cycloalcoyle en C3 à C7 ou un phényle mono-substitué par un membre choisi dans le groupe constitué par méthyle, trifluorométhyle, hydroxy, méthoxy, fluoro, chloro, bromo et iodo;

R4 est un hydrogène:

ou 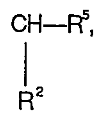

où R5 est un hydrogène; un alcoyle en C1 à C4; un hydroxy; ou un cycloalcoyle en C3 à C7; et

E est (1) $-Y-(CH_2)_n-R6$

où

Y est -NH- ou -O-;

n vaut de 0 à 5; et

R6 est un hydrogène; un hydroxy; un alcoyle en C1 à C4; un cycloalcoyle en C3 à C7; un aryle; un aryle substitué par jusqu'à 5 membres choisis indépendamment dans le groupe constitué par alcoyle en C1 à C6, trifluorométhyle, hydroxy, alcoxy en C1 à C4, amino, mono- ou di-alcoyle en C1 à C4-amino, et halo; amino; mono-, di- ou tri-alcoyle en C1 à C4-amino; guanidyle; un hétérocycle; ou un hétérocycle substitué par jusqu'à 5 membres choisis indépendamment dans le groupe constitué par alcoyle en C1 à C6, hydroxy, trifluorométhyle, alcoxy en C1 à C4, halo, aryle, aryl-alcoyle en C1 à C4, amino, et mono- ou di-alcoyle en C1 à C4-amino;

(2) 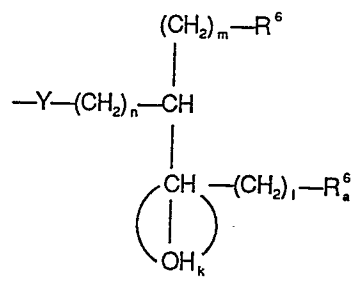

où

Y est tel que défini ci-dessus:

n vaut 0 ou 1;

k vaut 0 ou 1;

l vaut de 1 à 4;

m vaut de 1 à 4 et

$R^6$ et $R^6_a$ peuvent être semblables ou différents et ont la même signification que $R^6$ ci-dessus et $R^6_a$ peut en outre être

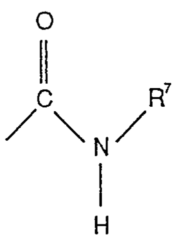

ou

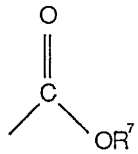

où R7 est un hydrogène ou un alcoyle en C1 à C3;

(3) 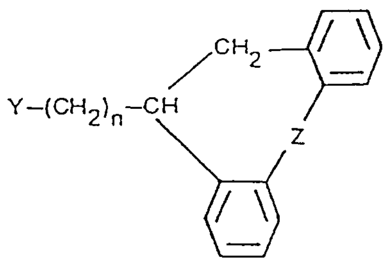

où
Y est tel que défini ci-dessus;
n vaut 0 ou 1; et
Z est

(a) 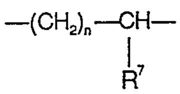

où
n vaut 0 ou 1; et
R7 est tel que défini ci-dessus: ou

(b) 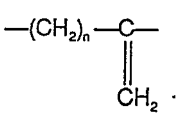

où
n vaut 0 ou 1;
où tous les atomes de carbone asymétriques ont une configuration S, sauf ceux qui sont dans les substituants B, D et E, qui peuvent avoir une configuration S ou R;
et d'un de ses sels pharmaceutiquement acceptables, ledit peptide comprenant de 5 à 7 acides aminés identifiés par I à VII, l'acide aminé (AA) I étant à l'extrémité C dudit peptide, à laquelle le substituant E est attaché, et l'acide aminé (AA) VII étant à l'extrémité N dudit peptide, à laquelle les substituants A-B-B-D sont attachés, à l'exception toutefois des peptides de formule (I) et de leurs sels, dans laquelle
$R^3$ est 2-propyle,
$R^4$ est méthyle,
E est

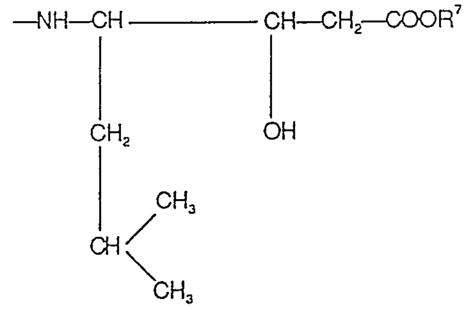

et B et D sont absents, comprenant les étapes consistant à:
(A) traiter l'ester ou amide désiré de l'acide aminé C-terminal (AA I) avec l'acide aminé adjacent suivant (AA II) dudit peptide, le groupe amino dudit acide aminé étant protégé par un groupe protecteur, en présence d'un agent de condensation, grâce à quoi il se forme un dipeptide des deux acides aminés (AA I et II);
(B) déprotéger le dipeptide formé dans l'étape (A) en enlevant le groupe protecteur du groupe amino de AA II;
(C) traiter le dipeptide de AA I et AA II avec AA III, dont le groupe amino est protégé par un groupe protecteur, en présence d'un agent de condensation, grâce à quoi il se forme un tripeptide de AA I, AA II et AA III;
(D) déprotéger le tripeptide formé dans l'étape (C) en enlevant le groupe protecteur du groupe amino de AA III;
(E) former un quadripeptide et jusqu'à un heptapeptide de AA I via AA IV, AA V, AA VI ou AA VII, en répétant le procédé de l'étape (C) en utilisant AA IV protégé jusqu'à AA VII protégé:
(F) déprotéger le quadripeptide à heptapeptide formé dans l'étape (E) pour donner le peptide de formule I où A est un hydrogène; et éventuellement
(G) traiter le quadripeptide à heptapeptide formé dans l'étape (F) avec

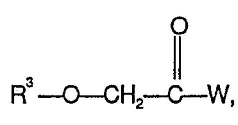, 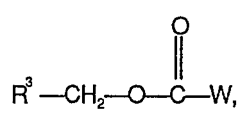,

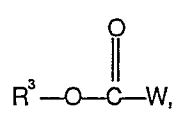, ou 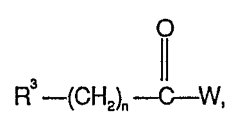,

où R3 et n sont tels que définis ci-dessus et W est un halogénure d'acide, un anhydride, ou un autre groupe activant de carboxyle, pour donner le peptide de formule I où A est différent d'un hydrogène et éventuellement
(H) traiter le quadripeptide à heptapeptide formé dans les étapes (F) ou (G) où l'on emploie un ester de AA I avec de l'hydrazine pour donner l'hydrazide correspondant, puis traiter ledit hydradzide avec un nitrite acide pour donner l'azide d'acyle correspondant, puis traiter ledit azide d'acyle avec le composé amine approprié pour donner le substituant E désiré dans le peptide de formule I; ledit procédé comprenant également, si nécessaire, la protection des substituants de chaîne latérale des acides aminés composants AA I à AA VII, la déprotection étant conduite comme étape finale; ledit procédé comprenant également toute combinaison des étapes exposées ci-dessus par laquelle les acides aminés I à VII et les substituants A, B, D et E sont assemblés dans n'importe quel ordre désiré pour préparer le peptide de formule I; et ledit procédé comprenant également l'emploi des étapes exposées ci-dessus dans une synthèse séquentielle en phase solide, grâce à quoi dans l'étape initiale le groupe carboxyle de l'acide aminé choisi est lié à un substrat de résine synthétique tandis que le groupe amino dudit acide aminé est protégé, puis l'enlèvement du groupe protecteur, les étapes successives étant telles qu'exposées ci-dessus, le peptide tel qu'assemblé étant attaché audit substrat de résine synthétique; suivi par une étape d'enlèvement du peptide de formule I dudit substrat de résine synthétique: (a) par clivage avec un acide fort pour donner E=OH; (b) par trans-estérification avec un alcanol en C1 à C4 pour donner E=O-alcolye en C1 à C4 (suivie par une hydrolyse pour donner E=OH); ou (c) par ammonolyse avec NH2R′ où R′ est un hydrogène ou un alcoyle en C1 à C4; et après enlèvement du peptide de formule I dudit substrat de résine synthétique par transestérification pour former son ester comme il est dit ci-dessus, éventuellement l'étape de traitement dudit ester selon les procédés décrits dans l'étape (H) ci-dessus pour donner le substituant E désiré dans le peptide de formule I; l'enlèvement des groupes protecteurs de chaîne latérale s'effectuant avant ou après l'enlèvement du peptide de formule I dudit substrat de résine synthétique.

2. Procédé de la revendication 1 pour préparer un peptide à partir

IBU - His-Pro-Phe-His-Sta-Leu-benzylamide;
IBU - His-Pro-Phe-His-Sta-Leu-2-phényléthylamide;
IBU - His-Pro-Phe-His-Sta-Leu-3-phénylpropylamide;
IBU - His-Pro-Phe-His-Sta-Leu-1,2-diphényléthylamide;
BOC - Phe-His-Sta-Leu-(+)-1,2-diphényléthylamide;
BOC - Phe-His-Sta-Leu-(−)-1,2-diphényléthylamide;
BOC - Phe-His-Sta-Leu-benzylamide;
BOC - Phe-His-Sta-Leu-(+)-α-phényléthylamide;
BOC - Phe-His-Sta-Leu-(−)-α-phényléthylamide;
BOC - Phe-His-Sta-Leu-(+)-α-naphthyléthylamide;
BOC - Phe-His-Sta-Leu-(−)-α-naphthyléthylamide;
BOC - Phe-His-Sta-Leu-p-chlorobenzylamide;
BOC - Phe-His-Sta-Leu-p-méthoxybenzylamide;
BOC - Phe-His-Sta-Leu-10,11-dihydro-5H-dibenzo-[a,d]-cycloheptèneamide;
BOC - Phe-His-Sta-Leu-D,L-thréo-1,2-diphényl-2-hydroxyéthylamide;
BOC - Phe-His-Sta-Leu-Sta;
BOC - Phe-His-AHPPA-Leu-benzylamide;
Acétyl - Phe-His-AHPPA-Leu-benzylamide;
BOC - Phe-His-Sta-Leu-(2-amidométhylpyridine);
BOC - Phe-His-Sta-Leu-(4-amidométhylpyridine);
BOC - Phe-His-Sta-Leu-(4-amido-1-benzylpipéridine);
BOC - Phe-His-Sta-Leu-[N-(3-amidopropyl)diéthanolamine];
BOC - Phe-His-AHPPA-Leu-(2-amidométhylpyridine);
BOC - Phe-His-ACHPA-Ile-(2-amidométhylpyridine);
IVA-His-D-Pro-Phe-His-ACHPA-Ile-(2-amidométhylpyridine).